# EUROPEAN PATENT APPLICATION

(11) **EP 3 492 467 A1**
(43) Date of publication of application: **05.06.2019**
(21) Application number: 17833600.4
(22) Date of filing: 28.07.2017
(51) Int. Cl.: C07D 455/06, C07D 471/04, C07D 491/048, C07D 491/056, C07D 498/04, C07D 513/04, A61K 31/5365, A61K 31/542, A61K 31/4375, A61P 31/20

(54) **ISOQUINOLINONE COMPOUND AND USE THEREOF IN PREPARATION OF ANTIVIRAL DRUGS**

(30) Priority: 29.07.2016 CN 201610626628; 16.08.2016 CN 201610671491
(71) Applicant: Ginkgo Pharma Co., Ltd., Suzhou, Jiangsu 215123 (CN)
(72) Inventor: CHEN, Li, Suzhou Jiangsu 215123 (CN); ZHAI, Peibin, Suzhou Jiangsu 215123 (CN); SHAO, Qing, Suzhou Jiangsu 215123 (CN); WU, Jin, Suzhou Jiangsu 215123 (CN); LI, Xiaowen, Suzhou Jiangsu 215123 (CN)
(74) Representative: Gulde & Partner
(86) International application number: PCT/CN2017/094946
(87) International publication number: WO 2018/019297

(57) **Abstract**

Disclosed is an isoquinolinone compound as shown in Formula (I) or a stereoisomer, pharmaceutically acceptable salt, solvate or crystal thereof, and a preparation method thereof, and use thereof in the preparation of drugs for treating or preventing viral infectious diseases caused by the hepatitis B virus (HBV) and other viruses, in particular, use thereof in drugs as HBV surface antigen inhibitors and HBV DNA production inhibitors. The compound has a significant activity in inhibiting hepatitis B surface antigen and hepatitis B DNA, and is possible to significantly improve the probability of curing hepatitis B by administration in combination with nucleoside drugs or other drugs in the future, and has good clinical application prospects.

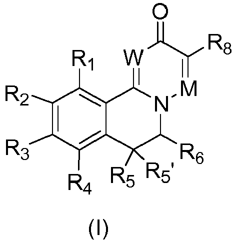

## Description

### Field of the invention

The present disclosure belongs to the field of medicinal and pharmaceutical chemistry, and specifically relates to a new type of isoquinolinone compounds or stereoisomers thereof, \to pharmaceutical compositions containing the foregoing isoquinolinone compounds or stereoisomers thereof and use of the pharmaceutical compositions as antiviral drugs, in particular, use of the pharmaceutical compositions in drugs as HBV Surface antigen inhibitors and HBV DNA production inhibitors for treating and/or preventing infection with hepatitis B virus, and in particular, to use thereof with TLR7 agonists and nucleoside drugs as pharmaceutical composition for curing hepatitis B.

### Background of the invention

There are approximately 350 million cases of chronic hepatitis B infection worldwide, and 780,000 people died of hepatitis B in 2011, among them, hepatitis B patients in China accounted for about one-third of the total number of hepatitis B patients worldwide. China currently spends more than 100 billion yuan a year on hepatitis B treatment, making it the world's largest market for hepatitis B drugs. Although hepatitis B vaccine has been widely used, hepatitis B patients in China are growing at an average rate of 2.5 million per year, and hepatitis B patients in the United States are also growing at a rate of 15.4%. About 25% of hepatitis B virus carriers are converted to chronic hepatitis B, and 10-30% of chronic hepatitis B develops into cirrhosis or liver cancer. Chronic hepatitis B is one of the main factors leading to cirrhosis.

Currently, there are 7 drugs approved by FDA for the treatment of hepatitis B, which are interferon-a, pegylated interferon-a, lamivudine, entecavir, telbivudine, adefovir dipivoxil and tenofovir. TAF (tenofovir alafenamide fumarate) has completed Phase III clinical trials, pending FDA approval. All of these drugs are not effective in curing hepatitis B and require long-term medication. Interferon drugs inhibit the virus's DNA and RNA by stimulating the body's immune system, and the use of interferon has less resistance and causes a certain loss of hepatitis B surface antigen and seroconversion, and has disadvantagesof low response rate, requirement of injection and serious side effects. Lamivudine and telbivudine easily cause drug resistance and cannot be taken for a long term. For example, 20% of patients taking lamivudine developed drug resistance in the first year, which reaches 70% in the second year. Adefovir dipivoxil gradually withdraws from first-line drugs due to tolerance and adverse reactions. At present, the first-line drugs recommended by WHO for treating hepatitis B are tenofovir disoproxil (TDF) and entecavir. In particular, there is no drug resistance after taking tenofovir disoproxil for 5 consecutive years, and the virus clearance rate was 95%-100%, and the side effects were small. All of these nucleoside drugs reduce hepatitis B virus by inhibiting the synthesis of DNAs of the virus, but have no effect onRNAs of the virus. Nucleoside drugs can only suppress the replication of hepatitis B virus, but can not cure hepatitis B. Therefore, a drug that simultaneously inhibits both DNAs and RNAs of the virus with a novel mechanism is required to cure hepatitis B. The newly developed core protein inhibitors can simultaneously inhibit DNAs and RNAsof the virus, and the therapeutic effect of single use can achieve similar to entecavir. Among them, NVR-3-778 is an effective capsid inhibitor, but there is no data on the disappearance of hepatitis B surface antigen (HBsAg). By analyzing existing data, capsid inhibitors are not able to cure hepatitis B because such compounds do not effectively act on cccDNA of hepatitis B, and there is no data to support that capsid inhibitors can shorten the half-life of cccDNA, and in phase II clinical studies, capsid inhibitors need to be combined with nucleoside drugs or interferons.

In the design of new drugs for the purpose of curing hepatitis B, the mechanism of the newly designed compounds is required to be the same as that of interferons, which are necessary to reactivate the body's own immune system and to recognise and remove infected liver cells by its own immune system, and thus completely cure hepatitis B. Hepatitis B surface antigen and other viral antigens secreted by hepatocytes from patients with chronic hepatitis B, through signal transduction systems, interfere with the immune system, and block the recognition of viruses by immune cells and further limit their antiviral function. In addition, persistent and excessive hepatitis B surface antigens can inactivate the immune system, delete T-cells, and damage performance functions. Hepatitis B surface antigen can also directly suppress the clearance function of immune cells on virus. Based on the above reasons, the development of drugs for inhibiting the secretion of hepatitis B surface antigen can effectively restore the functions of immune cells, reduce the pressure of the immune system, enable the immune system to recognize and remove infected liver cells, and achieve the goal of directly curing hepatitis B. In addition, the reduction of hepatitis B surface antigen is also a biological indicator of the improvement of chronic hepatitis B, and the disappearance of hepatitis B surface antigen and seroconversion indicate that hepatitis B has been functionally cured. At present, nucleoside drugs can not reduce hepatitis B surface antigen. It is necessary to design a new mechanism of action, which is used in combination with potent nucleoside drugs, to effectively remove hepatitis B surface antigen and DNA of the virus in the blood simultaneously, to activate and restore the body's immune function, and thus may eventually cure hepatitis B.

### Summary of the invention

The aim of the present disclosure is to provide a new type of isoquinolinone compounds with high activity for inhibiting the hepatitis B DNA and hepatitis B surface antigen. In addition, the structure of these compounds will block the pathway of P450 oxidation, increase the bioavailability thereof, and reduce the toxicity thereof. These highly active compounds will be combined with nucleoside drugs and TLR7 agonists, which may significantly improve the therapeutic effect and cure rate of hepatitis B in the clinic.

To achieve the above mentioned aims, the present disclosure employs the following technical solution:
An isoquinolinone compound of Formula (I) or a stereoisomer, pharmaceutically acceptable salt, solvate or crystal thereof, 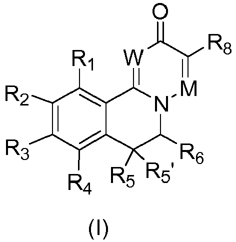 wherein
(1) R₁ is selected from H, deuterium, C₁₋₆ alkyl, cyano, halogen, carboxyl, ester, C₃₋₆ cycloalkyl, C₄₋₈ heterocycloalkyl and halogenated C₁₋₆ alkyl or C₆₋₁₀ aryl;
(2) R₂ is selected from halogen, C₁₋₃ alkoxy, deuterated C₁₋₃ alkoxy, C₁₋₆ alkyl, C₃₋₆ cycloalkyl, C₃₋₆ cycloalkyloxy, C₄₋₈ heterocycloalkyl C₁₋₆ alkyl, halogenated C₁₋₃ alkyloxy, halogenated C₃₋₆ cycloalkyl and C₃₋₆ cycloalkyl C₁₋₆ alkyl, or R₂ and R₃ are bonded by a carbon atom to form a ring;
(3) R₃ is (a) C₄₋₁₂ hydrocarbyl with a ring structure and/or an unsaturated bond, hydrogen in said C₄₋₁₂ hydrocarbyl is unsubstitued or substituted by one or more of deutrium, halogen, cyano, hydroxyl and sulphydryl, and said C₄₋₁₂ hydrocarbyl is uninterrupted by heteroatom or interrupted by one or more of O, S, NH, C=O, C=S, O=S=O, the heteroatom is selected from oxygen, sulphur or nitrogen; or (b) R₂ and R₃ are bonded by a carbon atom to form a ring;
(4) R₄ is selected from hydrogen, deuterium, halogen, cyano, ester or C₁₋₃ alkyl;
(5) R₅ and R₅' are independently selected from hydrogen, deuterium, halogen, methyl and methoxy, or R₅ and R₅' form a carbocyclic ring or a heterocyclic ring; or R₅ and R₆ form a carbocyclic ring or a heterocyclic ring;
(6) M is CH or N;
(7) R₆ is selected from C₁₋₆ alkyl, C₁₋₆ alkoxy C₁₋₆ alkyl, hydroxyl C₁₋₆ alkyl, aryl, halogenated C₁₋₆ alkyl, or C₃₋₆ cycloalkyl C₁₋₆ alkyl;
(8) W is N or CR₇, wherein R₇ is selected from hydrogen, deuterium, hydroxyl, halogen, C₁₋₃ alkyl, C₁₋₆ alkoxy, C₃₋₆ cycloalkyloxy, ester, carboxyl or cyano;
(9) R₈ is selected from carboxyl, ester, C₁₋₆ alkyl, C₃₋₆ cycloalkyl, C₁₋₆ alkyl alkynyl or C₃₋₆ cycloalkyl alkynyl; wherein, the alkyl portion of said ester is selected from C₁₋₆ alkyl, C₃₋₈ cycloalkyl, C₃₋₈ cycloalkyl alkynyl, C₁₋₆ alkyl alkynyl, benzyl, C₁₋₆ alkyl-C(O)O-C₁₋₃ alkyl and C₁₋₆ alkyl-OC(O)O-C₁₋₃ alkyl.

According to the present disclosure, hydrocarbyl interrupted by one or more of O, S, NH, C=O, C=S, O=S=O refers to that adjacent two carbon atoms of the hydrocarbyl group or the hydrocarbyl group and a carbon atom to which it is attached are interrupted by these atoms or groups, and there are no special restrictions on the position of interruption, provided that the bonding rules of the organic compound are satisfied. When it is interrupted by multiple atoms, these interrupting atoms or groups may be adjacent positioned or spaced apart. When there are multiple interrupting atoms or groups, they may be multiple identical atoms or groups, or they may be different atoms or groups. A new interrupting group, such as COO (ester), CONH (acylamino), SO₂NH (sulphonylamino) may be formed when two different interrupting atoms or groups are at adjacent positions. For example, a propyl group interrupted by one of O, S, NH, C=O, C=S, O=S=O may be OCH₂CH₂CH₃, CH₂OCH₂CH₃, CH₂SCH₂CH₃, CH₂NHCH₂CH₃, CH₂COCH₂CH₃, CH₂COCH₂CH₃, CH₂SO₂CH₂CH₃; a propyl group interrupted by two of O, S, NH, C=O, C=S, O=S=O may be CH₂COOCH₂CH₃, CH₂COCH₂OCH₃, CH₂CONHCH₂CH₃, CH₂C=OCHNHCH₃, CH₂SO₂NHCH₂CH₃, etc..

Further, in said (a), the ring structure is a 3- to 8- membered ring, more preferably a 3- to 6- membered ring; and, the unsaturated bond is a double bond or a triple bond.

Preferably, in said (a), the ring structure is a saturated ring.

Preferably, in said (a), the numbers of the ring structure and the unsaturated bond are 1 to 2, respectively.

Preferably, in said (a), there is a 3- to 8- membered saturated carbocyclic ring or a 3- to 8- membered saturated heterocyclic ring, and at least one heteroatom, or at least one double or triple bond.

More preferably, in said (a), at least two of said ring structure, said unsaturated bond and said heteroatom are simultaneously present.

According to one specific and preferred aspect of the present disclosure, said (a) is a group satisfying any one of the conditions described in the following:
a1) having and only having one said ring structure and one unsaturated carbon-carbon bond;
a2) having both said ring structure and 1 - 3 heteroatoms, and at least one of the heteroatoms is oxygen, which is connected to a benzene ring in said Formula (I) through a single bond;
a3) having both said unsaturated bond and 1 - 3 heteroatoms, wherein the unsaturated bond is a carbon-carbon doulbe bond, a carbon-carbon triple bond or a carbon-oxygen doulbe bond, and when the unsaturated bond is a carbon-carbon doulbe bond or a carbon-carbon triple bond, one end thereof is preferably connected to the benzene ring in said Formula (I) through a single bond.

According to one specific aspect of the present disclosure, R₃ is selected from C₅₋₁₁ bicycloalkyl, C₃₋₆ cycloalkyl alkynyl, C₃₋₆ cycloalkyl alkenyl, C₁₋₃ alkoxy C₁₋₆ alkyl alkynyl, C₁₋₃ alkoxy C₁₋₆ alkyl alkenyl and C₄₋₈ heterocycloalkyl; or
R₃ is R_{A}-O-, R_{A} is selected from C₃₋₈ cycloalkyl; C₅₋₁₁ bicycloalkyl; deuterated C₁₋₆ alkyl; C₄₋₈ heterocycloalkyl; C₁₋₆ alkyl carbonyl C₁₋₆ alkyl; deuterated C₁₋₃ alkoxyC₁₋₆ alkyl; C₁₋₃ alkoxy C₃₋₈ cycloalkyl; C₁₋₃ alkoxy C₃₋₈ cycloalkyl C₁₋₆ alkyl; C₃₋₈ heterocycloalkyl ; C₁₋₃ alkoxy C₁₋₆ alkyl, wherein alkyl is substituted by C₃₋₈ cycloalkyl or C₄₋₈ heterocycloalkyl, and a heteroatom in heterocycloalkyl is selected from oxygen, sulphur or nitrogen, when R_{A} is C₁₋₃ alkoxy C₁₋₆ alkyl, R₅ and R₅' are independently selected from deuterium, fluorine, chlorine, hydroxyl and cyano, and W is N or CR₇, wherein R₇ is selected from deuterium, fluorine, chlorine, hydroxyl, and cyano.

According to one preferred aspect of the present disclosure, R₃ is selected from C₃₋₈ cycloalkoxy, C₃₋₈ heterocycloalkyloxy, C₁₋₃ alkoxy C₃₋₈ cycloalkoxy, C₁₋₃ alkoxy C₃₋₈ cycloalkyl C₁₋₆ alkoxy, C₃₋₈ heterocycloalkyl, C₁₋₃ alkoxy C₂₋₉ alkenyl, C₁₋₃ alkoxy C₂₋₉ alkynyl, C₃₋₈ cycloalkyl C₂₋₉ alkenyl, C₃₋₈ cycloalkyl C₂₋₉ alkynyl.

According to the present disclosure, R₂ is selected from C₁₋₃ alkoxy, halogen, C₃₋₆ cycloalkyl, benzyl.

According to one specific aspect of the present disclosure, R₆ is selected from methyl, ethyl, isopropyl, butyl, isobutyl, methoxy methyl, methoxy ethyl, methoxy isopropyl, methoxy butyl, methoxy isobutyl, ethoxy methyl, ethoxy ethyl, ethoxy isopropyl, ethoxy butyl, ethoxy isobutyl, hydroxyl methyl, hydroxyl ethyl, hydroxyl isopropyl, hydroxyl butyl and hydroxyl isobutyl.

According to the present disclosure, except for the active hydrogens, all other hydrogen atoms can be independently replaced by deutrium.

According to the present disclosure, typical isoquinolinone compounds are as follows:

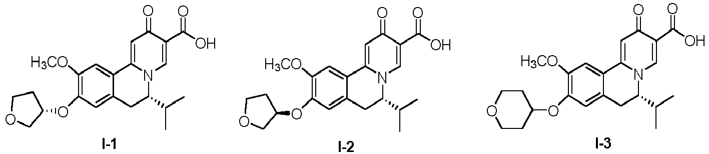

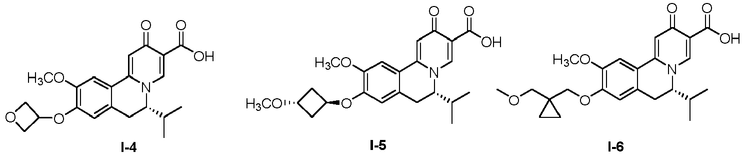

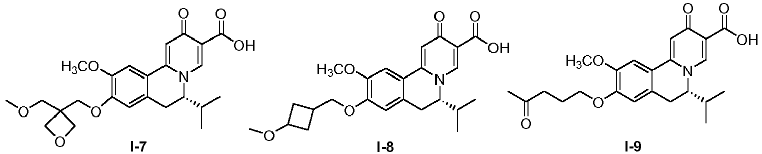

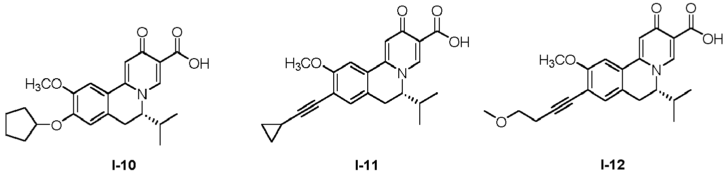

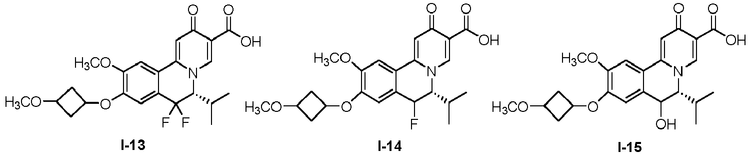

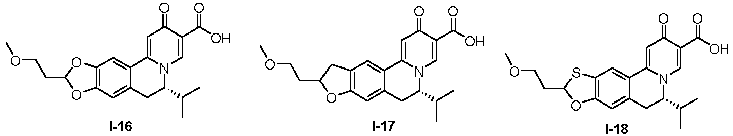

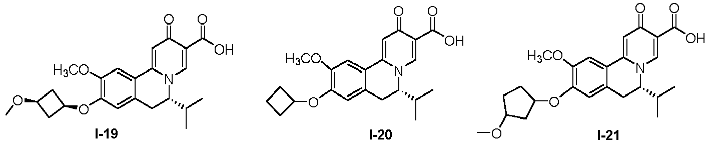

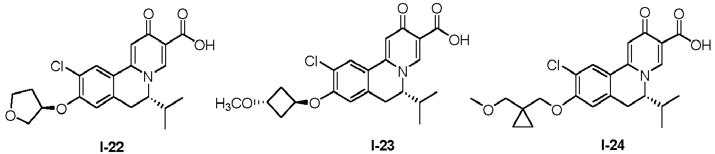

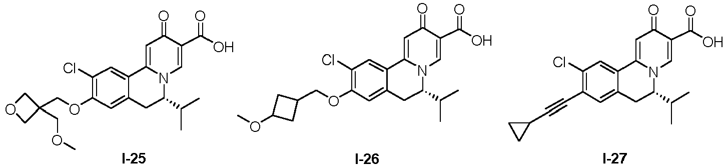

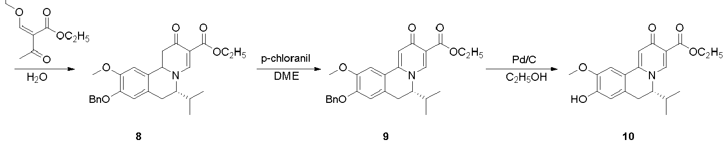

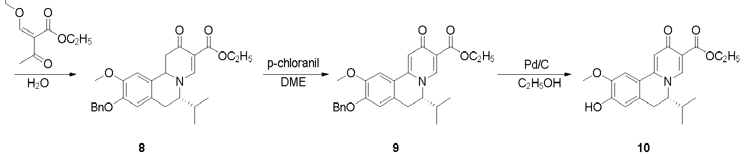

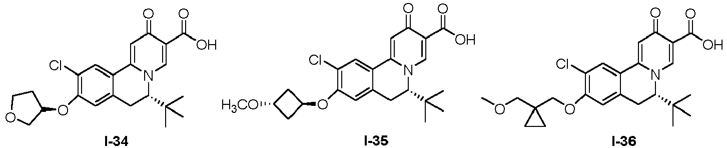

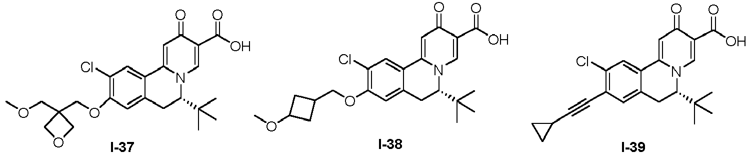

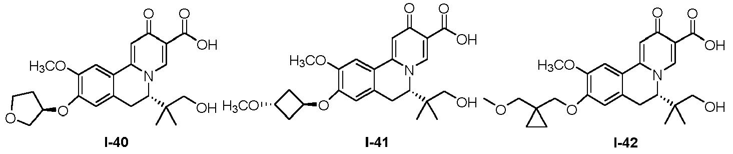

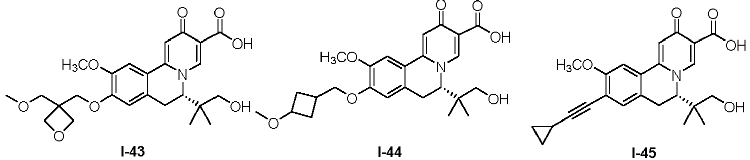

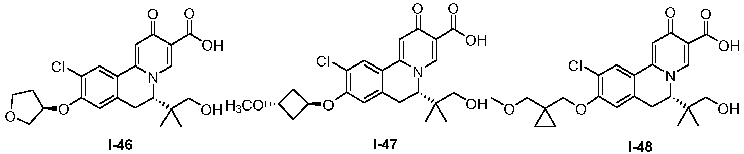

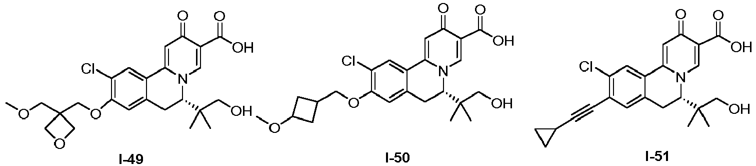

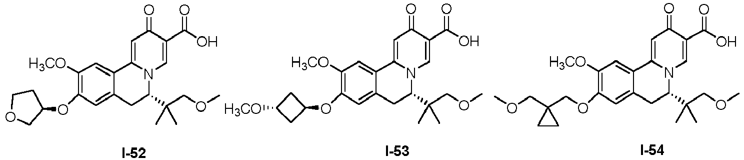

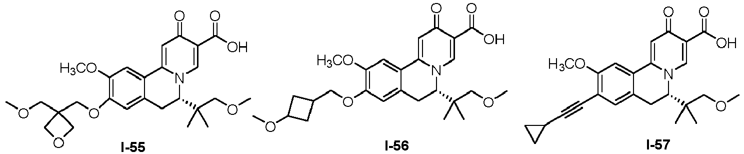

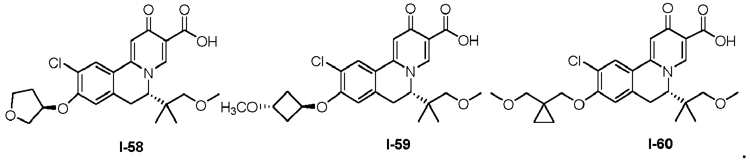

This disclosure also provides an intermediate for preparing the isoquinolinone compound shown in Formula (1) or the stereoisomer, the pharmaceutically acceptable salt, the solvate or the crystal thereof of the present disclosure, and the intermediate is shown in Formula (II): 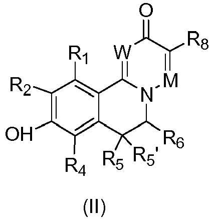 in Formula (II), R₁, R₂, R₄, R₅, R₅', R₆, R₈, W and N are as defined as above.

According to a specific and preferred aspect of the present disclosure, the intermediate shown in Formula (II) is Compound 10 or an isomer or a racemate thereof.

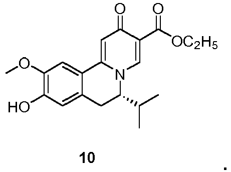

This disclosure further provides a process for preparing the isoquinolinone compound shown in Formula (I) or the stereoisomer, the pharmaceutically acceptable salt, the solvate or the crystal thereof of the present disclosure (hereinafter collectively referred to as the compound of the present disclosure), the process comprises employing the intermediate shown in the following Formula (II): 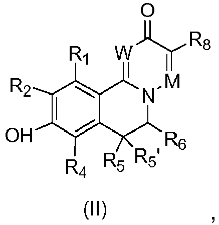 in Formula (II), R₁, R₂, R₄, R₅, R₅', R₆, R₈, W and N are as defined as above.

Further, the method comprises reacting the intermediate shown in Formula (II) with R_{A}OH, R_{A}OMs or R_{A}Br, wherein, when the reactant is R_{A}OH, the reaction is carried out using a Mitsunobu reactionin the presence of a dehydrating agent of triphenylphosphine and/or diisopropyl azodicarboxylate; when the reactant is R_{A}OMs or R_{A}Br, the reaction is an SN₂ reaction, and carried out in the presence of a base of potassium carbonate and/or cesium carbonate and a catalytic amount of KI.

According to a specific aspect of the present disclosure, the intermediate shown in Formula (II) is Compound 10 or an isomer or a racemate thereof.

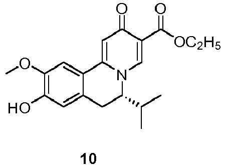

The present disclosure also provides a pharmaceutical composition comprising the isoquinolinone compound shown in Formula (1) or the stereoisomer, the pharmaceutically acceptable salt, the solvate or the crystal thereof of the present disclosure, and a pharmaceutically acceptable carrier or excipient.

Preferably, the pharmaceutical composition is an antiviral pharmaceutical composition, wherein it further contains one or more therapeutic agents selected from : nucleoside drugs, ribavirin, interferons, HBV capsid inhibitors, cccDNA formation inhibitors, cccDNA epigenetic modifiers or hepatitis B RNAi drugs and TLR7 agonists.

The present disclosure also relates to use of the isoquinolinone compound shown in Formula (1) or the stereoisomer, the pharmaceutically acceptable salt, the solvate or the crystal thereof of the present disclosure or a combination thereof with one or more therapeutic agents selected from nucleoside drugs, ribavirin, interferons, HBV capsid inhibitors, cccDNA formation inhibitors, cccDNA epigenetic modifiers, hepatitis B RNAi drugs or TLR7 agonists in the preparation of a medicament for preventing and/or treating virus infection diseases, and/or in the preparation of HVB surface antigen inhibitors and HVB DNA production inhibitors, the virus infection includes infection with HBV or HDV.

The present disclosure also provides use of the pharmaceutical composition in preparation of a medicament for treating or preventing hepatitis B and hepatitis B virus infection, and a method for preventing or slowing the disease of a patient infected with hepatitis B and hepatitis B virus using the pharmaceutical composition.

The pharmaceutical composition according to the disclosure is preferably present in a therapeutically effective amount.

A pharmaceutically acceptable carrier in the above pharmaceutical composition is, such as a pharmaceutically acceptable diluent, excipient, filler, binder, disintegrant, absorption enhancer, surfactant, lubricant, fragrance, sweeteners, etc..

The drug prepared by using the compound of the present disclosure as an active ingredient may be in various forms such as a tablet, a powder, a capsule, a granule, an oral solution, and an injection preparation.The dosage form of the pharmaceutical composition is preferably a tablet, capsule or injection.

The above various dosage forms of the drug can be prepared by a conventional method in the pharmaceutical field.

The present disclosure also provides use of the compound of the present disclosure in the preparation of a medicament for the prevention or treatment of a viral infection, preferably the viral infection is an HBV infection.

The pharmaceutical composition of the present disclosure may be composed of the following ratio:

| | |
|---|---|
| Compound of the present disclosure | 5-95% |
| Lactose | 1-60% |
| Starch | 0-20% |
| Microcrystalline cellulose | 1-40% |
| Carboxymethyl starch sodium | 1-5% |
| Polyethylene glycol (PEG6000) | 0-10% |
| Magnesium stearate | 1-5% |

Due to the implementation of the above technical solutions, the present disclosure has the following advantages compared with the prior art:
The present disclosure provides novel isoquinolinone compounds, which have strong inhibition on hepatitis B DNA activity, EC50 thereof being less than 5 nanomole, and have strong activity for inhibiting hepatitis B surface antigen, EC50 thereof being about 10 nanomole. In addition, such compounds have excellent pharmacokinetic properties. Further, these compounds of the disclosure will block the pathway of P450 oxidation, increase the bioavailability of the compounds, and reduce the toxicity of the compounds. These highly active compounds will be administrated in combination with nucleoside compounds and TLR7 agonists, which may significantly improve the therapeutic effect and cure rate of hepatitis B in the clinic.

### Definition of Terms

Unless otherwise defined, all technical and scientific terms used herein have the same meaning as commonly understood by one of ordinary skill in the art to which this disclosure belongs. The term "stereoisomer" refers to an isomer produced by the different arrangement of atoms in a molecule in space. These include cis and trans isomers, enantiomers and conformational isomers. All stereoisomers are within the scope of the present disclosure. The compound of the present disclosure may be a single stereoisomer or a mixture of other isomers such as a racemate, or a mixture of all other stereoisomers.

The term "salt" refers to a pharmaceutically acceptable salt formed by a compound of the present disclosure with an acid, which may be an organic or inorganic acid, specifically selected from phosphoric acid, sulfuric acid, hydrochloric acid, hydrobromic acid, citric acid, maleic acid, malonic acid, mandelic acid, succinic acid, fumaric acid, acetic acid, lactic acid, nitric acid, sulfonic acid, p-toluenesulfonic acid, malic acid, methanesulfonic acid or analogues thereof.

The term "solvate" refers to a form of a compound of the present disclosure that forms a solid or liquid complex by coordination with a solvent molecule. Hydrates are a special form of solvates in which coordination occurs with water. Within the scope of the present disclosure, the solvate is preferably a hydrate.

The term "crystal" refers to the various solid forms formed by the compounds described herein, including crystalline forms and amorphous forms.

The term "hydrocarbyl" refers to a saturated or unsaturated linear, branched or cyclic substituent consisting essentially of carbon and hydrogen. It has preferably 1 to 20 carbon atoms, more preferably 1 to 12 carbon atoms. The term "alkyl" refers to a linear, branched or cyclic saturated hydrocarbyl group. The alkyl group specifically includes methyl, ethyl, n-propyl, isopropyl, cyclopropyl, n-butyl, isobutyl, tert-butyl, cyclobutyl, n-pentyl, isopentyl, neopentyl, cyclohexyl, n-hexyl, isohexyl, 2,2,-dimethylbutyl and 2,3-dimethylbutyl, 16-alkyl, 18-alkyl. The term "C₁₋₂₀ alkyl" means a linear, branched or cyclic saturated hydrocarbyl group containing 1 to 20 carbon atoms. Alkyl groups include substituted and unsubstituted alkyl groups. When an alkyl group is substituted, the substituent may substitute at any available point of attachment, and the substitution may be mono-substitution or poly-substitution. The substituent is independently selected from alkyl, alkenyl, alkynyl, alkoxy, alkylthio, alkylamino, deuterum, halogen, thiol, hydroxy, nitro, carboxy, ester, cyano, cycloalkyl, aryl, heteroaryl, cycloalkoxy, heterocycloalkoxy, cycloalkylthio, oxo. The substituent is usually placed before the alkyl group when naming, for example, C₁₋₃ alkoxy C₃₋₈ cycloalkyl C₁₋₆ alkyl means C₁₋₆ alkyl is substituted by C₃₋₈ cycloalkyl, and C₃₋₈ cycloalkyl is further substituted by C₁₋₃ alkoxy. For example: the structural formula of methoxycyclobutylmethyl is:

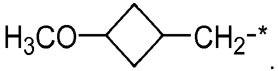

The terms "alkenyl" and "alkynyl" mean, respectively, a linear, branched or cyclic unsaturated hydrocarbyl group containing a double bond and a triple bond, preferably containing 2 to 20 carbon atoms, more preferably 2 to 12 carbon atoms. Alkenyl and alkynyl include substituted and unsubstituted alkenyl and alkynyl. When substituted, the substituent may substitute at any available point of attachment, and the substitution may be mono-substitution or poly-substitution. The substituent is independently selected from alkyl, alkenyl, alkynyl, alkoxy, alkylthio, alkylamino, deuterium, halogen, thiol, hydroxy, nitro, carboxy, ester, cyano, cycloalkyl, aryl, heteroaryl, cycloalkoxy, heterocycloalkoxy, cycloalkylthio, oxo. The substituent is usually placed before the alkenyl or alkynyl group when naming.

The term "cycloalkyl" refers to a saturated and/or partially unsaturated monocyclic or polycyclic cyclohydrocarbyl group. A single ring may include 3 to 10 carbon atoms. Nonlimiting examples of monocyclic cycloalkyl groups include cyclopropyl, cyclobutyl, cyclopentyl, cyclopentenyl, cyclohexyl, cyclohexenyl, cyclohexadienyl, cycloheptyl and the like. Polycyclic cycloalkyl groups include spiro, fused, and bridged cycloalkyl groups. The cycloalkyl group includes a substituent or no substituent. The substituent is selected from one or more substituents including, but not limited to, the following groups: alkyl, cycloalkyl, alkoxy, halogen, carboxyl, ester, amino, amide, hydroxy, cyano, nitro, aryl, heteroaryl.

The term "aryl" refers to a 6- to 10- membered all-carbon monocyclic or polycyclic aromatic group, including phenyl, naphthyl, biphenyl, and the like. The aryl group can be substituted and unsubstituted. The substituent is independently selected from alkyl, cycloalkyl (cyclopropyl, cyclobutyl, cyclopentyl, etc.), alkenyl, alkynyl, azide, amino, deuterium, alkoxy, alkylthio, alkylamino, halogen, thiol, hydroxy, nitro, heterocycloalkyl, aryl, heteroaryl, cycloalkoxy, heterocycloalkoxy, cycloalkylthio, heterocycloalkylthio, alkylsilyl and so on.

The term "heteroaryl" refers to a radical of a heteroaromatic system containing 1 to 10 heteroatoms. Heteroatoms include oxygen, sulfur, nitrogen, phosphorus, and the like. Wherein monoheterocyclic groups include, but not limited to, furan, thiophene, pyrrole, thiazole, imidazole, 1,2,3-triazole, 1,2,4-triazole, 1,2,3-thiadiazole, oxazole, 1,2,4-oxadiazole, 1,3,4-oxadiazole, pyridine, pyrimidine, pyridazine, pyrazine, tetrahydrofuran, tetrahydropyrrole, piperidine, piperazine, morpholine, isoxazolin and the like. Fused heterocyclic groups include, but not limited to, quinoline, isoquinoline, indole, benzofuran, benzothiophene, purine, acridine, carbazole, fluorene, chromenone, fluorenone, quinoxaline, 3, 4-dihydronaphthalen, dibenzofuran, hydrogenated dibenzofuran, benzoxazolyl, and the like. Heteroaryl groups can be substituted and unsubstituted. The substituent is independently selected from alkyl, cycloalkyl (cyclopropyl, cyclobutyl, and cyclopentyl, etc.), alkenyl, alkynyl, azide, amino, deuterium, alkoxy, alkylthio, alkylamino, halogen, thiol, hydroxy, nitro, heterocycloalkyl, aryl, heteroaryl, cycloalkoxy, heterocycloalkoxy, cycloalkylthio, heterocyclethio, alkylsilyl and the like.

The term "halogen" means fluorine, chlorine, bromine, iodine, preferably fluorine, chlorine, bromine.

The term "deuterium" is an isotope of hydrogen, the atomic mass is twice that of the latter, and the binding to carbon is stronger. "Deuterated" and "deuterium" means that hydrogen is replaced with deuterium at the specified position. A "deuterated substituent" is a substituent in which at least one hydrogen is replaced by deuterium enriched at a specified percentage.

The term "haloalkyl" refers to an alkyl group substituted with at least one halogen atom.

The term "heterocyclic group" means a cyclic group containing at least one hetero atom, wherein the hetero atom is nitrogen, oxygen, sulfur, or the like. The heterocyclic groups include monoheterocyclic groups and a polyheterocyclic groups.

### Detailed description of examplary embodiments

The following embodiments are intended to provide a fuller understanding of the present disclosure, but are not intended to limit the disclosure in any way. The structures of all compounds were determined by ¹H NMR or MS.

The compound names used in the embodiments are abbreviated as follows:
DCM: dichloromethane
EtOAc: ethyl acetate
THF: tetrahydrofuran
DME: 1,2-dimethoxyethane
Dioxane: 1,4-dioxane
Pd₂(dba)₃: tris(dibenzylideneacetone)dipalladium
Xantphos: 4,5-Bis(diphenylphosphino)-9,9-dimethylxanthene
tBuONa: sodium tert-butoxide
POCl₃: phosphorus oxychloride
NH₄OAc: ammonium acetate
NaBH₃CN: sodium cyanoborohydride
p-chloranil: tetrachlorobenzoquinone
MsCI: methanesulfonyl chloride
Et₃N: triethylamine
BnBr: benzyl bromide
DIBAL-H: diisobutylaluminium hydride
PPh₃: triphenylphosphine
DEAD: diethyl azodicarboxylate
PhN(OTf)₂: N-Phenylbis(trifluoromethanesulphonimide)
B(OMe)₃: trimethyl borate
nBuLi: n-butyllithium
Pd(dppf)Cl₂: [1,1'-Bis(diphenylphosphino)ferrocene]dichloropalladium(II)

The present disclosure will be further described below in conjunction with specific embodiments:

### Preparation of Compound 10:

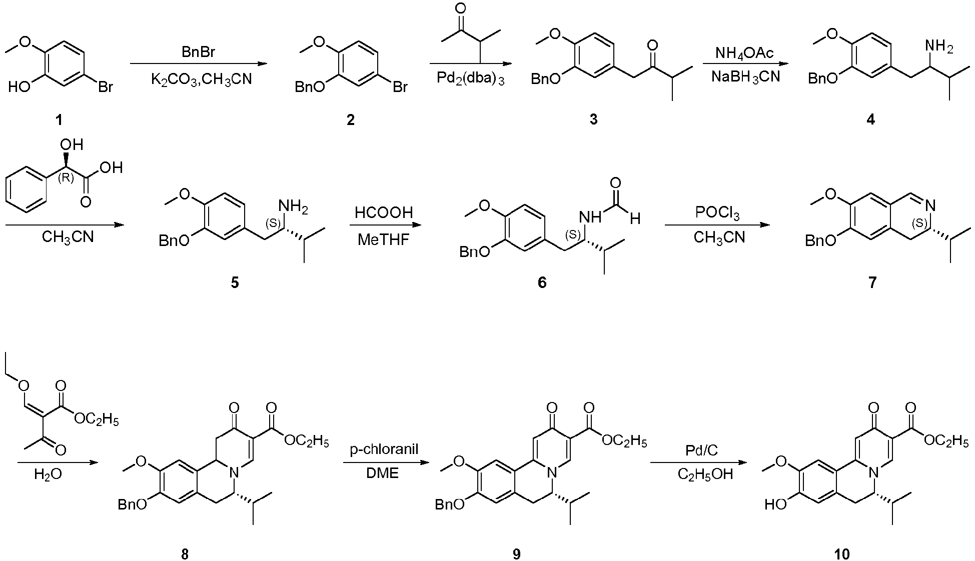

Preparation of Compound 2: A mixture of compound 1 (350 g, 1.72 mol), BnBr (352 g, 2.06 mol) and potassium carbonate (K₂CO₃, 368 g, 2.66 mol) was refluxed in a mixed solvent of acetonitrile (4.5 L) and acetone (4.3 L) for 18h under nitrogen protection. After cooling, the mixture was filtered and the filtrate was concentrated. The residue was diluted with EtOAc and washed with water and saturated salt solution, then dried over anhydrous sodium sulfate, filtered and concentrated. The residue was purified by column chromatography to give Compound 2 (437 g, 86.4% yield). ¹H NMR (CDCl₃, 300 MHz) δ: 7.33 - 7.48 (m, 5 H), 7.04 - 7.08 (m, 2 H), 7.77 (s, 1H), 5.12 (s, 2 H), 3.86 (s, 3 H).

Preparation of Compound 3: Compound 2 (330 g, 1.12 mol) was dissolved in THF (2.8 L). To this solution was added 3-methyl-2-butanone (145 g, 1.69 mol), Pd₂(dba)₃ (10 g, 11.2 mmol), Xantphos (19 g, 33.6 mmol) and tBuONa (162 g, 1.69 mol). The reaction system was replaced with nitrogen three times and stirred at 55 °C for 6h under nitrogen protection. The mixture was spun to dryness, and to the residue was added water (800 mL) and EtOAc (1000 mL) and stirred for 30min. The mixture stood to layer, and the aqueous phase was extracted with EtOAc (500 mL × 2). The organic phases were combined and washed with saturated salt solution, dried over anhydrous sodium sulfate, filtered and spin-dried. The residue was purified by column chromatography to give Compound 3 (187 g, 55.6% yield). ¹H NMR (CDCl₃, 300 MHz) δ: 7.46 - 7.28 (m, 5 H), 6.88 - 6.76 (m, 3 H), 5.15 (s, 2 H), 3.89 (s, 3 H), 3.63 (s, 2 H), 2.68 - 2.63 (m, 1 H), 1.05 (d, *J* = 6.9 Hz, 6 H).

Preparation of Compound 4: Compound 3 (180 g, 0.6 mol) and ammonium acetate (465 g, 6.0 mol) were added in methyl alcohol (MeOH, 1.4 L) and cooled in ice bath. Then NaBH₃CN (30 g, 0.48 mol) was added in portions. The mixture was stirred at room temperature for 10 min, then at 50 °C for 21 hours. The mixture was cooled and treated with water (490 mL) and 10N sodium hydroxide solution (aq. NaOH, 120 mL, 1.2 mol), and stirred at room temperature for 1 hour. The mixture was extracted with DCM (500 mL × 3), the combined organic layers were combined and washed with saturated salt solution, dried over anhydrous sodium sulfate and spin-dried to give Compound 4 (176 g, 97.4% yield). ¹H NMR (CDCl₃, 300 MHz) δ: 7.46 - 7.28 (m, 5 H), 6.86 - 6.76 (m, 3 H), 5.16 (s, 2 H), 3.88 (s, 3 H), 2.76 - 2.72 (m, 2 H), 2.37 - 2.29 (m, 1 H), 1.63 - 1.54 (m, 3 H), 0.94 (d, *J* = 6.9 Hz, 6 H).

Preparation of Compound 5: Compound 4 (3.0 g, 10 mmol) was stirred in acetonitrile (84 mL) for 30 min, then R-madenlic acid (0.84 g, 5.5 mmol) was added and the mixture was stirred at 55°C overnight. The mixture was cooled to room temperature and filtered, and the solid was washed with acetonitrile (16 mL) and dried to give 1.6 g solid. The solid was added into water (8 mL) and 1M K₂CO₃ solution (4.2 mL, 4.2 mmol) was added. The mixture was stirred at room temperature for 2 h to give a clean solution. The system was added with DCM (15 mL) and salt (0.4 g), and the organic phase was separated and washed with saturated salt solution, dried and spin-dried to give Compound 5 (0.80 g, 26.6% yield, 98% ee).

Preparation of Compound 6: Methanoic acid (86.2 g, 1.87 mol) was added to a solution of Compound 5 (75 g, 0.25 mol) in methyl tetrahydrofuran (670 mL) and the mixture was refluxed for 12 h. The mixture was cooled and concentrated. The residue was dissolved in EtOAc and washed with water and saturated salt solution, then dried over anhydrous sodium sulfate and spin-dried to give Compound 6 crude (70 g). The crude product was used directly in the next step.

Preparation of Compound 7: Compound 6 (70 g, 0.21 mol) was dissolved in acetonitrile (622 mL). POCl₃ (41 g, 0.27 mol) was added and the mixture was stirred at 90 °C for 2 hours. After the reaction finished, the mixture was cooled and the reaction was quenched by addition of water in ice water bath, then the mixture was concentrated and added with 1N sodium hydroxide to adjust pH = 10. The mixture was extracted with DCM (200 mL × 3). The organic phases were combined and washed with saturated salt solution, dried and concentrated. The residue was purified by column chromatography to give Compound 7 (56 g, 86.1% yield). ¹H NMR (CDCl₃, 300 MHz) δ: 8.39 (s, 1 H), 7.47 - 7.34 (m, 5 H), 6.92 (s, 1 H), 6.74 (s, 1 H), 5.22 (s, 2 H), 3.93 (s, 3 H), 3.42- 3.39 (m, 1 H), 2.67- 2.63 (m, 2 H), 2.11 - 2.09 (m, 1 H), 1.11 - 1.01 (m, 6H); ESI-MS *m*/*z* 310.2 (M+H)⁺.

Preparation of Compound 8: Compound 7 (12.5 g, 40.5 mmol) and ethyl 2-ethoxymethylacetoacetate (22.7 g, 0.12 mol) were added in water (118 mL) and the mixture was stirred at 85 °C for 29 h. After the reaction finished, the mixture was cooled down to room temperature, and extracted with EtOAc. The organic phases were combined and washed with saturated salt solution, dried and concentrated to give 24.5 g crude product.

Preparation of Compound 9: Compound 8 (50 g, 0.11 mol) and tetrachlorobenzoquinone (27.6 g, 0.11 mol) were dissolved in DME (470 ml) and stirred at 70 °C for 3h. After the reaction finished, the reaction solution was filtered, and the filtrate was concentrated and poured into water and extracted with DCM (200 mL × 3), the organic layer were combined and washed with saturated salt solution, dried and concentrated to give a crude product, which was purified by column chromatography to give Compound 9 (23.1 g, 46.2% yield). ¹H NMR (CDCl₃, 300 MHz) δ: 8.17 (s, 1 H), 7.46 - 7.34 (m, 5 H), 7.20 (s, 1 H), 6.92 (s, 1 H), 6.72 (s, 1 H), 5.22 (s, 2 H), 4.41 (q, 2 H), 3.94 (s, 3 H), 3.72 - 2.69 (m, 1 H), 3.29 - 3.23 (m, 1 H), 2.98 - 2.93 (m, 1 H), 1.80 - 1.72 (m, 1 H), 1.41 (m, 3 H), 0.89 - 0.78 (m, 6 H).

Preparation of Compound 10: Pd/C (6 g) was added to a solution of Compound 9 (23.0 g, 51.4 mmol) in ethanol (250 mL) and the mixture was stirred under hydrogen atmosphere for 15 h. After the reaction finished, the mixture was filtered and spin-dried. The residue was recrystallized from DCM to give Compound 10 (10.0 g, 54.0% yield). ¹H NMR (CDCl₃, 300 MHz): δ 8.28 (s, 1 H), 7.17 (s, 1 H), 7.07 (s, 1 H), 6.87 (s, 1 H), 4.41 (q, *J* = 6.9 Hz, 2 H), 3.94 (s, 3 H), 3.85 - 3.81 (m, 1 H), 3.33 - 3.26 (m, 1 H), 3.04 - 2.97 (m, 1 H), 1.84 - 1.76 (m, 1 H), 1.41 (t, *J* = 6.9 Hz, 3 H), 0.94 - 0.92 (m, 3 H), 0.84 - 0.82 (m, 3 H); ESI-MS *m*/*z* 358.1 (M+H)⁺.

Using the same process, the racemic Compounds 7-rac and 10-rac may be obtained from unresolved starting materials.

### Embodiment 1: Preparation of 6-isopropyl-10-methoxy-2-oxo-9-(((S)-tetrahydrofuran-3-yl)oxy)-6,7-dihydro-2H-pyrido[2,1-α]isoquinoline-3-carboxylic acid (I-1-rac)

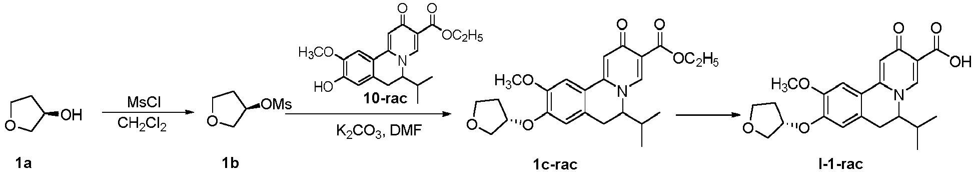

Preparation of Compound 1b: In an ice-water bath, Compound 1a (0.20 g, 2.27 mmol) was dissolved in DCM (6 mL) and the solution was added with TEA (Et₃N, 1 mL, 7.19 mmol) and with MsCI (0.39 g, 3.42 mmol) slowly. The mixture was stirred at room temperature for 3 h and spin-dried. The residue was dissolved in EtOAc (60 mL) and washed with water, saturated sodium carbonate and saturated salt solution, then dried over anhydrous sodium sulfate, filtered and spin-dried to give 0.36g crude product.

Preparation of Compound 1c-rac: A mixture of Compound 10-rac (100 mg, 0.28 mmol), Compound 1b (93 mg, 0.56 mmol) and K₂CO₃ (116 mg, 0.84 mmol) in 5mL N, N-dimethyl formamide (DMF) was stirred at 90 °C for 18 h. After the reaction finished, the mixture was poured into water and extracted with EtOAc (40 mL × 3). The organic phases were combined and washed with water and saturated salt solution, dried over anhydrous sodium sulfate, filtered and spin-dried to give 0.128 g crude product.

Preparation of Compound I-1-rac: To a solution of Compound 1c-rac (0.128 g, 0.30 mmol) in THF (5 mL) was added 1N aq. NaOH (1.8 mL, 1.80 mmol) and the mixture was reacted at room temperature for 18h. After the reaction finished, the solution was added with 1N hydrochloric acid (HCl) to adjust pH = 1-2, and extracted with DCM (30 mL×3). The organic layers were combined and dried over anhydrous sodium sulfate, filtered and spin-dried to give a crude product. The crude product was recrystallized from EtOAc and tert-butyl methyl ether (TBME) to give Compound I-1-rac (34 mg, 28.3% yield). ¹H NMR (400MHz, CDCl₃) δ: 8.44 (s, 1H), 7.19 (s, 1H), 7.05 (s, 1H), 6.70-6.67 (m, 1H), 5.06-5.00 (m, 1H), 4.06-4.00 (m, 3H), 3.97-3.92 (m, 1H), 3.91 (s, 3H), 3.88-3.84 (m, 1H), 3.36-3.31 (m, 1H),3.21 (s, 3H), 3.08-3.06 (m, 1H), 3.04 (s, 1H), 3.03-3.02 (m, 1H), 2.27-2.18 (m, 2H), 1.86-1.77 (m, 1H), 0.94-0.93 (d, *J* = 6.4 Hz, 3H), 0.84-0.82 (d, *J* = 6.8 Hz, 3H); ESI-MS *m*/*z* 400.2 (M+H)⁺.

### Embodiment 2: Preparation of (S)-6-isopropyl-10-methoxy-2-oxo-9-(((R)-tetrahydrofuran-3-yl)oxy)-6,7-dihydro-2H-pyrido[2,1-α]isoquinoline-3-carboxylic acid (I-2)

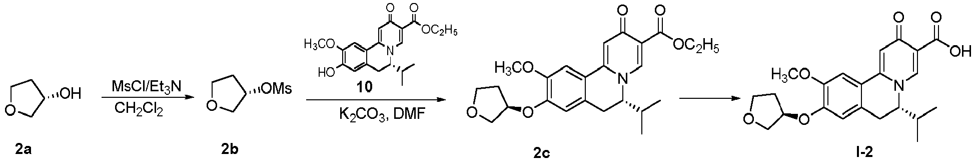

Preparation of Compound 2b: Compound 2a (0.20 g, 2.27 mmol) was dissolved in DCM (5 mL) and the solution was added with TEA (1 mL, 7.19 mmol) and with MsCI (0.39 g, 3.41 mmol) slowly. The mixture was stirred at room temperature for 3 h and spin-dried. The residue was dissolved in EtOAc (50 mL) and washed with water, saturated sodium carbonate and saturated salt solution, then dried over anhydrous sodium sulfate, filtered and spin-dried to give Compound 2b crude product (0.36g).

Preparation of Compound 2c: A mixture of Compound 10 (0.16 g, 0.45 mmol), Compound 2b (0.149 g, 0.90 mmol) was in 3mL DMF and then the solution was added with K₂CO₃ (0.186 g, 1.34 mmol) and reacted at 90 °C for 18 h. After the reaction finished, the mixture was poured into water and extracted with EtOAc (40 mL×3). The ethyl acetate layers were combined and washed with water and saturated salt solution, then dried over anhydrous sodium sulfate, filtered and spin-dried to give Compound 2c crude product (0.287 g).

Preparation of Compound I-2: Compound 2c (0.287 g, 0.67 mmol) was dissolved in 5 mL THF, and the solution was added with 1N aq. NaOH (4.0 mL, 4.0 mmol) and reacted at room temperature for 18h. After the reaction finished, the solution was added with 1N HCI to adjust pH = 1-2 and extracted with DCM (30 mL×3). The organic layers were combined and dried over anhydrous sodium sulfate, filtered and spin-dried to give a crude product. The crude product was recrystallized from EtOAc and TBME to give Compound I-2 (0.113 g, 63.4% yield). ¹H NMR (400MHz, CDCl₃) δ: 8.47 (s, 1H), 7.20 (s, 1H), 7.07 (s, 1H), 6.69 (s, 1H), 5.09-5.01 (m, 1H), 4.10-3.99 (m, 3H), 3.92 (s, 3H), 3.85-3.40 (m, 2H), 3.36 (dd, *J*₁ = 16.4 Hz, *J*₂ = 5.6 Hz, 1H), 3.11-3.04 (m, 1H), 2.29-2.23 (m, 2H), 1.87-1.78 (m, 1H), 0.95 (d, *J* = 6.8Hz, 3H), 0.85 (d, *J* = 6.8Hz, 3H); ESI-MS *m*/*z* 400.2 (M+H)⁺.

### Embodiment 3: Preparation of 6-isopropyl-10-methoxy-2-oxo-9-(((R)-tetrahydrofuran-3-yl)oxy)-6,7-dihydro-2H-pyrido[2,1-α]isoquinoline-3-carboxylic acid (I-2-rac)

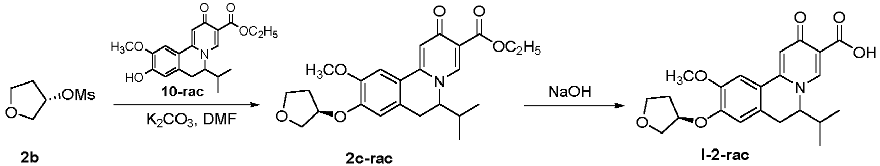

Compound I-2-rac was obtained according to the process of Embodiment 2. ¹H NMR (400MHz, CDCl₃) δ: 8.47 (s, 1H), 7.20 (s, 1H), 7.07 (s, 1H), 6.69 (s, 1H), 5.09-5.01 (m, 1H), 4.10-3.99 (m, 3H), 3.92 (s, 3H), 3.85-3.40 (m, 2H), 3.36 (dd, *J*₁ = 16.4 Hz, *J*₂ = 5.6 Hz, 1H), 3.11-3.04 (m, 1H), 2.29-2.23 (m, 2H), 1.87-1.78 (m, 1H), 0.95 (d, *J* = 6.8Hz, 3H), 0.85 (d, *J* = 6.8Hz, 3H); ESI-MS *m*/*z* 400.2 (M+H)⁺.

### Embodiment 4: Preparation of 6-isopropyl-10-methoxy-2-oxo-9-((tetrahydro-2H-pyran-4-yl)oxy)-6,7-dihydro-2H-pyrido[2,1-α]isoquinoline-3-carboxylic acid (I-3-rac)

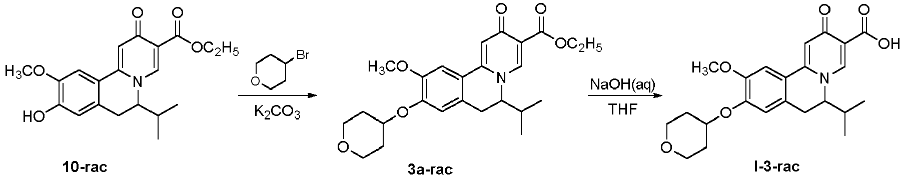

Preparation of Compound 3a-rac: Compound 10-rac (100 mg, 0.28 mmol) and 4-bromotetrahydropyran (94 mg, 0.56 mmol) were dissolved in 5mL DMF, and then the solution was added with K₂CO₃ (78 mg, 0.56 mmol) and reacted at 90°C for 40 h. After the reaction finished, the mixture was poured into water and extracted with EtOAc (40 mL×3). The ethyl acetate layers were combined and washed with water and saturated salt solution, then dried over anhydrous sodium sulfate and filtered, and the filtrate was vacuum concentrated to give a crude product, which was purified by a preparation plate to give Compound 3a-rac (65 mg, 56.1% yield).

Preparation of Compound I-3-rac: Compound 3a-rac (65 mg, 0.15 mmol) was dissolved in THF (5 mL), and the solution was added with 1N aq. NaOH (1.0 mL, 1.0 mmol) and the mixture was reacted at room temperature for 18h. After the reaction finished, the solution was added with 1N HCl to adjust pH = 1-2 and extracted with DCM (30 mL×3). The organic layers were combined and dried over anhydrous sodium sulfate, filtered and spin-dried to give a crude product, which was purified by a preparation plate to give Compound I-3-rac (2 mg, 3.2% yield). ¹H NMR (400MHz, CDCl₃) δ:15.94 (s, 1H), 8.44 (s, 1H), 7.19 (s, 1H), 7.06 (s, 1H), 6.77 (s, 1H), 4.57 (m, 2H), 4.03-4.02 (m, 2H), 3.92 (s, 3H), 3.87-3.84 (m, 1H), 3.61-3.55 (m, 2H), 3.35-3.30 (m, 1H), 3.07-3.03 (m, 1H), 2.07-2.04 (m, 1H), 1.81-1.93 (m, 4H), 0.94-0.93 (d, *J* = 6.4Hz, 3H), 0.84-0.82 (d, *J* = 6.8Hz, 3H); ESI-MS *m*/*z* 414.2 (M+H)⁺.

### Embodiment 5: Preparation of 6-isopropyl-10-methoxy-9-(oxetan-3-yloxy)-2-oxo-6,7-dihydro-2H-pyrido[2,1-α]isoquinoline-3-carboxylic acid (I-4-rac)

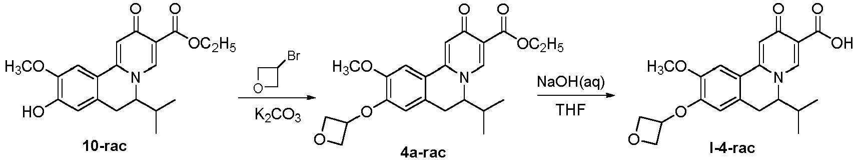

Preparation of Compound 4a-rac: Compound 10-rac (100 mg, 0.28 mmol) and 3-bromo-oxetane (75 mg, 0.56 mmol) were dissolved in DMF (5mL), and then the solution was added with K₂CO₃ (78 mg, 0.56 mmol) and stirred at 90 °C for 40 h. After the reaction finished, the mixture was poured into water and extracted with EtOAc (40 mL×3). The ethyl acetate layers were combined and washed with water and saturated salt solution, then dried over anhydrous sodium sulfate, filtered and spin-dried to give a crude product, which was purified by a preparation plate to give Compound 4a-rac (51 mg, 42.9 % yield).

Preparation of Compound I-4-rac: Compound 4a-rac (50 mg, 0.12 mmol) was dissolved in THF (5 mL), and the solution was added with 1N aq. NaOH (1.0 mL, 1.0 mmol) and the mixture was reacted at room temperature for 18h. After the reaction finished, the solution was added with 1N HCl to adjust pH = 1-2 and extracted with DCM (30 mL×3). The organic layers were combined and dried over anhydrous sodium sulfate, filtered and spin-dried to give a crude product, which was purified by a preparation plate to give Compound I-4-rac (7 mg, 15.1% yield). ¹H NMR (400MHz, CDCl₃) δ:15.87 (s, 1H), 8.45 (s, 1H), 7.21 (s, 1H), 7.06 (s 1H), 6.33 (s, 1H), 4.98 (s, 2H), 4.82 (s, 2H), 3.94 (s, 5H), 3.30 (s, 1H), 3.03 (s, 1H), 1.78 (s, 1H), 0.92 (s, 3H), 0.82 (s, 3H); ESI-MS *m*/*z* 386.2 (M+H)⁺.

### Embodiment 6: Preparation of (S)-6-isopropyl-10-methoxy-9-((1R,3S)-3-methoxycyclobutoxy)-2-oxo-6,7-dihydro-2H-pyrido[2,1-α]isoquinoline-3-carboxylic acid (I-5)

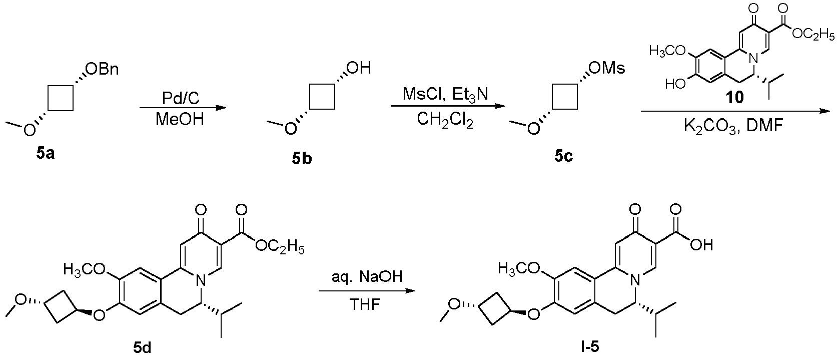

Preparation of Compound 5b: Compound 5a (0.70 g, 3.64 mmol) was dissolved in 100 mL MeOH, and the solution was added with Pd/C (0.12 g) and 1 drop of concentrated HCl, replaced with hydrogen three times, and hydrogenated at room temperature for 18h. The solution was filtered, and spin-dried to give 0.43 g Compound 5b crude product.

Preparation of Compound 5c: Compound 5b (0.43 g, 4.22 mmol) was dissolved in 8 mL DCM, and the solution was added with TEA (1.5 mL, 8.63 mmol) and with MsCI (0.72 g, 6.32 mmol) slowly. The mixture was stirred at room temperature for 3 h and spin-dried to give a crude product. The crude product was dissolved in EtOAc (50 mL) and washed with water, saturated sodium carbonate and saturated salt solution, then dried over anhydrous sodium sulfate, filtered and spin-dried to give 0.70 g Compound 5c crude product.

Preparation of Compound 5d: Compound 10 (0.160 g, 0.45 mmol) and Compound 5c (0.161 g, 0.90 mmol) were dissolved in DMF (5mL), and then the solution was added with K₂CO₃ (0.185 g, 1.34 mmol) and stirred at 90 °C for 18 h. The mixture was poured into water and extracted with EtOAc (30 mL×4). The ethyl acetate layers were combined and washed with water and saturated salt solution, then dried over anhydrous sodium sulfate, filtered and spin-dried to give 0.21 g oily product, i.e., Compound 5d.

Preparation of Compound I-5: Compound 5d (0.210 g, 0.48 mmol) was dissolved in THF (5 mL), and the solution was added 1N aq. NaOH (2.85 mL, 2.85 mmol) and reacted at room temperature for 18h. After the reaction finished, the solution was added with 1N HCI to adjust pH = 1-2 and extracted with DCM (30 mL×3). The organic layers were combined and dried over anhydrous sodium sulfate, filtered and spin-dried to give a crude product, which was purified by a preparation plate to give Compound I-5 (64 mg, 32.2% yield). ¹H NMR (400MHz, CDCl₃) δ: 8.46 (s, 1H), 7.18 (s, 1H), 7.07 (s, 1H), 6.54 (s, 1H), 5.00-4.90 (m, 1H), 4.20-4.13 (m, 1H), 3.94 (s, 3H), 3.91-3.84 (m, 1H), 3.52-3.34 (m, 1H), 3.31 (s, 3H), 2.59-2.46 (m, 1H), 2.59-2.48 (m, 4H), 1.88-1.78 (m, 1H), 0.95 (d, *J* = 6.8Hz, 3H), 0.84 (d, *J* = 6.8Hz, 3H); ESI-MS *m*/*z* 414.2 (M+H)⁺.

### Embodiment 7: Preparation of 6-isopropyl-10-methoxy-9-((1R,3S)-3-methoxycyclobutoxy)-2-oxo-6,7-dihydro-2H-pyrido[2,1-α]isoquinoline-3-carboxylic acid (I-5-rac)

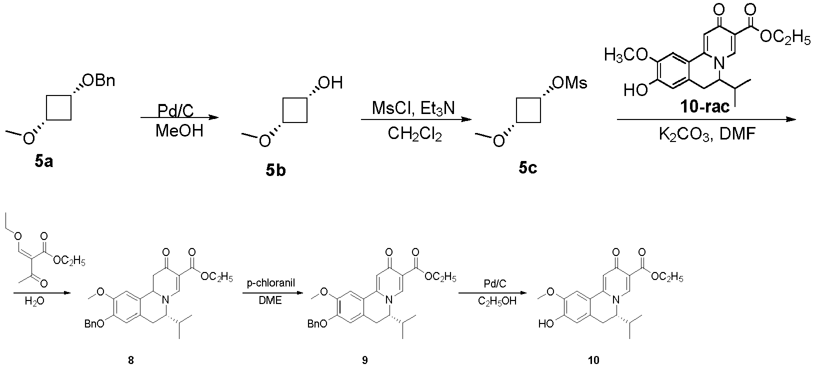

Compound I-5-rac was obtained according to the process of Embodiment 6. ¹H NMR (400MHz, CDCl₃) δ: 8.46 (s, 1H), 7.18 (s, 1H), 7.07 (s, 1H), 6.54 (s, 1H), 5.00-4.90 (m, 1H), 4.20-4.13 (m, 1H), 3.94 (s, 3H), 3.91-3.84 (m, 1H), 3.52-3.34 (m, 1H), 3.31 (s, 3H), 2.59-2.46 (m, 1H), 2.59-2.48 (m, 4H), 1.88-1.78 (m, 1H), 0.95 (d, *J* = 6.8Hz, 3H), 0.84 (d, *J* = 6.8Hz, 3H); ESI-MS *m*/*z* 414.2 (M+H)⁺.

### Embodiment 8: Preparation of (S)-6-isopropyl-10-methoxy-9-((1-(methoxymethyl)cyclopropyl)methoxy)-2-oxo-6,7-dihydro-2H-pyrido[2,1-α]isoquinoline-3-carboxylic acid (I-6)

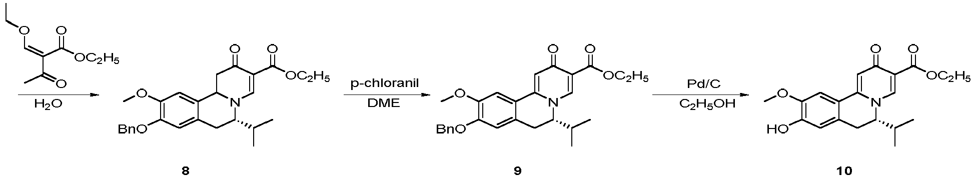

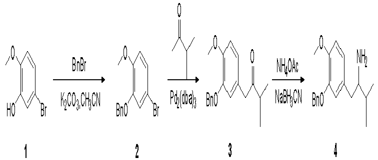

Preparation of Compound 6b: A mixture of Compound 6a (0.50 g, 4.9 mmol), K₂CO₃ (1.02 g, 7.4 mmol), methyl iodide (CH₃I, 1.04 g, 7.4 mmol) and 4-fluorobenzeneboronic acid (69 mg, 0.49 mmol) in DMF (5 mL) was stirred at room temperature for 16h. The reaction liquid was added with 30mL water and extracted with EtOAc (30 mL×3). The organic phases were combined and washed with saturated salt solution for once, dried over anhydrous sodium sulfate, and spin-dried to give 200 mg Compound 6b crude product, which was directly used in the next step. ¹H NMR (400 MHz, CDCl₃) δ: 3.54 (d, *J* = 5.2Hz, 2H), 3.38 (s, 2H), 3.37 (s, 3H), 2.51 (t, *J* = 5.2Hz, 1H), 0.50-0.55 (m, 4H).

Preparation of Compound 6c: Compound 6b (0.20 g, 1.72 mmol) was dissolved in DCM (10 mL), and the solution was cooled in an ice-water bath. To the solution was added TEA (0.35 g, 3.44 mmol) and MsCI (0.30 g, 2.59 mmol). The mixture was reacted at room temperature for 2 h, then diluted with DCM (20 mL) and washed with 1N HCl (10 mL). The aqueous phase was extracted with DCM (20 mL×2). The organic phases were combined and washed with saturated salt solution, dried over anhydrous sodium sulfate, and spin-dried to give 0.30g yellow oil, i.e., Compound 6c.

Preparation of Compound 6d: Compound 10 (0.15 g, 0.42 mmol), Compound 6c (0.30 g, 1.54 mmol) and K₂CO₃ (0.12 g, 0.84 mmol) were stirred in 5mL DMF at 90 °C for 16 h. After the reaction finished, the mixture was cooled to room temperature, diluted with 30 mL water and extracted with EtOAc (20 mL×2). The organic layers were combined and washed with saturated salt solution, dried over anhydrous sodium sulfate and concentrated to give 0.18g Compound 6d crude product, which was directly used in the next step.

Preparation of Compound I-6: To a solution of Compound 6d (0.18 g, 0.40 mmol) in MeOH (10 mL) was added 1N aq. NaOH (1.6 mL, 1.60 mmol) and the mixture was reacted at 50 °C for 1.5h. After the reaction finished, the solution was added with 1N HCl to adjust pH = 1-2 and extracted with DCM (20 mL×3). The organic layers were combined and dried over anhydrous sodium sulfate, filtered and spin-dried to give a crude product, which was purified by a preparation plate to give Compound I-6 (26 mg, 15.2% yield). ¹H NMR (400MHz, CDCl₃) δ: 8.47 (s, 1H), 7.17 (s, 1H), 7.07 (s, 1H), 6.76(s, 1H), 3.98 (m, 2H), 3.91 (s, 3H), 3.88 (m, 1H), 3.40 (m, 2H), 3.35 (s, 3H), 3.29-3.34 (m, 1H), 3.02-3.06 (m, 1H), 1.82 (m, 1H), 0.92 (d, *J* = 6.8Hz, 3H), 0.81 (d, *J* = 6.8Hz, 3H), 0.67 (m, 2H), 0.64 (m, 2H); ESI-MS *m*/*z* 428.2 (M+H)⁺.

### Embodiment 9: Preparation of 6-isopropyl-10-methoxy-9-((1-(methoxymethyl)cyclopropyl)methoxy)-2-oxo-6,7-dihydro-2H-pyrido[2,1-α]isoquinoline-3-carboxylic acid (I-6-rac)

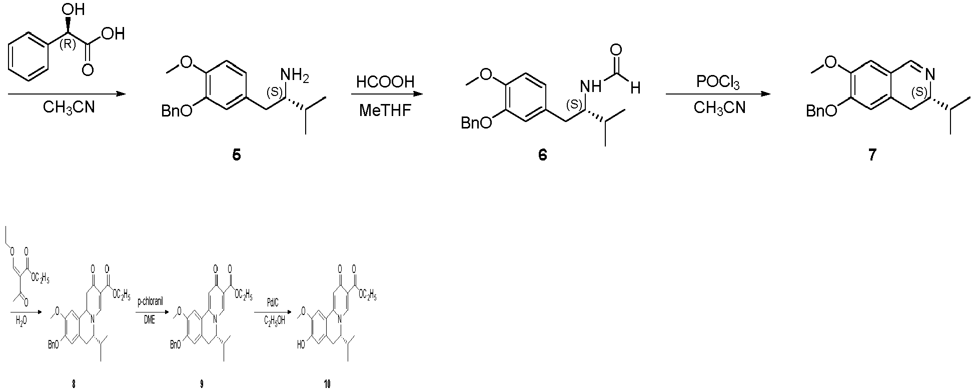

Compound I-6-rac was obtained according to the process of Embodiment 8. ¹H NMR (400MHz, CDCl₃) δ: 8.47 (s, 1H), 7.17 (s, 1H), 7.07 (s, 1H), 6.76(s, 1H), 3.98 (m, 2H), 3.91 (s, 3H), 3.88 (m, 1H), 3.40 (m, 2H), 3.35 (s, 3H), 3.29-3.34 (m, 1H), 3.02-3.06 (m, 1H), 1.82 (m, 1H), 0.92 (d, *J* = 6.8Hz, 3H), 0.81 (d, *J* = 6.8Hz, 3H), 0.67 (m, 2H), 0.64 (m, 2H); ESI-MS *m*/*z* 428.2 (M+H)⁺.

### Embodiment 10: Preparation of (S)-6-isopropyl-10-methoxy-9-((3-(methoxymethyl)oxetan-3-yl)methoxy)-2-oxo-6,7-dihydro-2H-pyrido[2,1-α]isoquinoline-3-carboxylic acid (I-7)

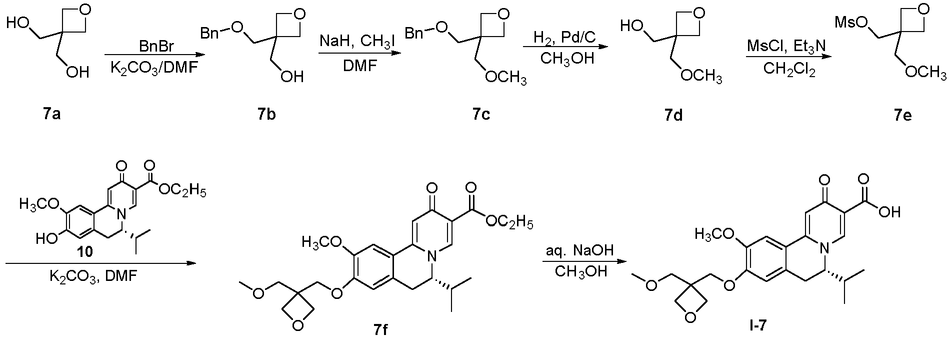

Preparation of Compound 7b: A mxiture of Compound 7a (0.27 g, 2.3 mmol), anhydrous K₂CO₃ (0.48 g, 3.5 mmol), BnBr (0.59 g, 3.5 mmol) and 4-fluorobenzeneboronic acid (32 mg, 0.23 mmol) in 2 mL DMF was stirred at room temperature for 2 days. The reaction liquid was added with 20mL water and extracted with EtOAc (20 mL×3). The organic phases were combined and washed with saturated salt solution, then dried over anhydrous sodium sulfate and spin-dried to give a crude product, which was purified by column chromatography to give 0.28 g oil, i.e., Compound 7b. ¹H NMR (400MHz, CDCl₃) δ: 7.31-7.36 (m, 5H), 4.56 (s, 2H), 4.48 (d, *J* = 6.4Hz, 2H), 4.42 (d, *J* = 6.0Hz, 2H), 3.93 (d, *J* = 4.4Hz, 2H), 3.80 (s, 2H), 2.34 (t, *J* = 5.2Hz, 1H).

Preparation of Compound 7c: Compound 7b (0.28 g, 1.35 mmol) was dissolved in dry DMF (5 mL), and the solution was cooled in an ice-water bath. The solution was added with NaH (65 mg, 2.7 mmol) and stirred for 40 min in an ice-water bath. Then CH₃I (0.48 g, 3.38 mmol) was added and the mixture was stirred for another 5h in an ice-water bath. The reaction was quenched by addition of 30 mL water and the solution was extracted with EtOAc (20 mL×3). The organic phases were combined and washed with saturated salt solution, dried over anhydrous sodium sulfate and spin-dried to give a crude product, which was seperated by column chromatography to give colorless oily Compound 7c (0.18 g, 60.1% yield). ¹H NMR (400MHz, CDCl₃) δ: 7.29-7.36 (m, 5H), 4.56 (s, 2H), 4.48 (m, 4H), 3.67 (s, 2H), 3.62 (s, 2H), 3.38 (s, 3H).

Preparation of Compound 7d: Compound 7c (0.18 g, 0.81 mmol) was dissolved in 10 mL MeOH, and the solution was added with Pd/C (18 mg). The reaction proceeded under H₂ atmosphere for 16h. Then 1 drop of concentrated HCl was added and the mixture was stirred under H₂ atmosphere for 4h. The solution was filtered, and the filtrate was spin-dried and seperated by column chromatography to give a colorless liquid, i.e., Compound 7d (0.11 g, 100% yield). ¹H NMR (400MHz, CDCl₃) δ: 4.49 (d, *J* = 6.4Hz, 2H), 4.43 (d, *J* = 6.0Hz, 2H), 3.94 (m, 2H), 3.74 (s, 2H), 3.40 (s, 3H).

Preparation of Compound 7e: Compound 7d (0.11 g, 0.83 mmol) was dissolved in 10 mL DCM, and the solution was cooled in an ice-water bath. To the solution was added TEA (0.168 g, 1.66 mmol) and MsCI (0.143 g, 1.25 mmol). The mixture was reacted at room temperature for 2 h, then diluted with 10 mL DCM and washed with 1N HCl. The aqueous phase was extracted with DCM (20 mL×3). The organic phases were combined and washed with saturated salt solution, dried over anhydrous sodium sulfate and spin-dried to give 0.13 g yellow oil, i.e., Compound 7e.

Preparation of Compound 7f: A mixture of Compound 10 (0.15 g, 0.42 mmol), Compound 7e (0.13 g, 0.63 mmol) and anhydrous K₂CO₃ (0.12 g, 0.84 mmol) in 5mL DMF was stirred at 90 °C for 16 h. After the reaction finished, the mixture was cooled to room temperature, diluted with 30 mL water and extracted with EtOAc (20 mL×3). The organic phases were combined and washed with saturated salt solution, dried over anhydrous sodium sulfate and concentrated to give a crude product, which was seperated by TLC to give a white solid, i.e., Compound 7f (0.16 g, 76.2% yield).

Preparation of Compound I-7: To a solution of Compound 7f (0.16 g, 0.32 mmol) in MeOH (10 mL) was added 1N aq. NaOH (1.3 mL, 1.30 mmol) and the mixture was reacted at 50 °C for 2h. After the reaction finished, the solution was added with 1N HCl to adjust pH = 1-2 and extracted with DCM (20 mL×3). The organic layers were combined and dried over anhydrous sodium sulfate, filtered and spin-dried to give a white solid Compound I-7 (94 mg, 66.2% yield). ¹H NMR (400MHz, CDCl₃) δ:8.45 (s, 1H), 7.18 (s, 1H), 7.06 (s, 1H), 6.82 (s, 1H), 4.56-4.63 (m, 4H), 4.28 (m, 2H), 3.90 (s, 3H), 3.86 (m, 1H), 3.77 (m, 2H), 3.40 (s, 3H), 3.32-3.36 (m, 1H), 3.05-3.09 (m, 1H), 1.84 (m, 1H), 0.95 (d, *J* = 6.8Hz, 3H), 0.83 (d, *J* = 6.8Hz, 3H); ESI-MS *m*/*z* 444.2 (M+H)⁺.

### Embodiment 11: Preparation of 6-isopropyl-10-methoxy-9-((3-(methoxymethyl)oxetan-3-yl)methoxy)-2-oxo-6,7-dihydro-2H-pyrido[2,1-α]isoquinoline-3-carboxylic acid (I-7-rac)

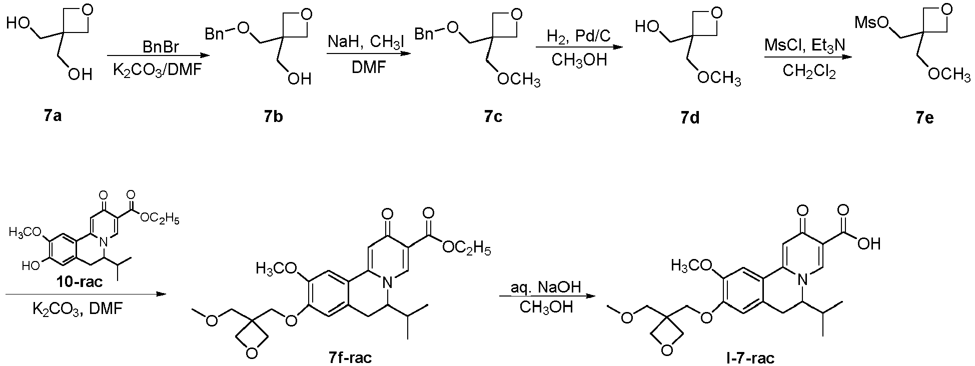

Compound I-7-rac was obtained according to the porcess of Embodiment 10. ¹H NMR (400MHz, CDCl₃) δ: 8.45 (s, 1H), 7.18 (s, 1H), 7.06 (s, 1H), 6.82(s, 1H), 4.56-4.63 (m, 4H), 4.28 (m, 2H), 3.90 (s, 3H), 3.86 (m, 1H), 3.77 (m, 2H), 3.40 (s, 3H), 3.32-3.36 (m, 1H), 3.05-3.09 (m, 1H), 1.84 (m, 1H), 0.95 (d, *J* = 6.8Hz, 3H), 0.83 (d, *J* = 6.8Hz, 3H); ESI-MS *m*/*z* 444.2 (M+H)⁺.

### Embodiment 12: Preparation of (S)-6-isopropyl-10-methoxy-9-((3-methoxycyclobutyl)methoxy)-2-oxo-6,7-dihydro-2H- pyrido[2,1-α]isoquinoline-3-carboxylic acid (I-8)

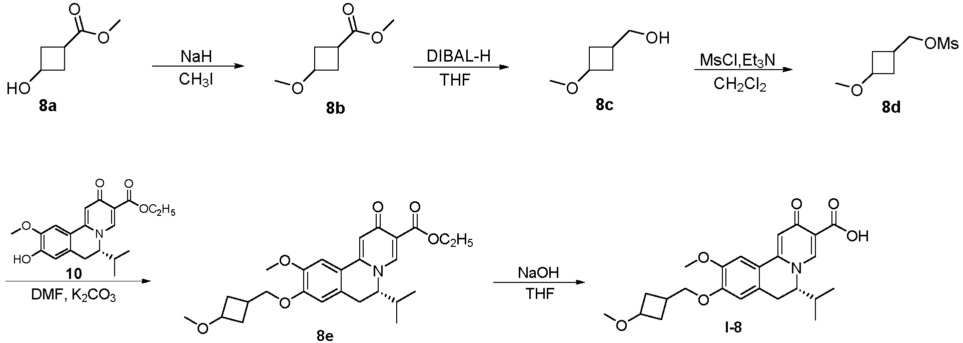

Preparation of Compound 8b: Compound 8a (5.0 g, 38 mmol) was dissolved in dry 50 mL THF, and the solution was added with NaH (1.84 g, 76 mmol) in portions under the condition of an ice-water bath, and stirred for 30 min after addition finished. Then CH₃I (2.87 mL, 46 mmol) was dropwise added slowly.The mixture was stirred and reacted at room temperature for 3h. TLC showed reaction completion. The reaction liquid was poured into saturated NH₄Cl solution to quench and extracted with EtOAc (30 mL×3). The organic phases were combined and dried over anhydrous sodium sulfate and purified by silica gel column chromatography (EtOAc: petroleum ether (PE)=1: 5) to give Compound 8b (1.90 g, 34.2% yield). ¹H NMR (400MHz, CDCl₃) δ: 3.81-3.73 (m, 1H), 3.66 (s, 3H), 3.21 (s, 3H), 2.66-2.58 (m, 1H), 2.51-2.45 (m, 2H), 2.21-2.13 (m, 2H).

Preparation of Compound 8c: Compound 8b (1.90 g, 13 mmol) was dissolved in anhydrous THF (20 mL) and replaced with nitrogen three times in an ice-water bath. The mixture was dropwise added with a solution of 1.5M DIBAL-H in THF (26.4 mL, 39 mmol). After addition finished, the mixture was stirred overnight. The reaction was monitored by TLC until the reaction finished. The reaction was quenched by addition of 2N HCl (20 mL) and the solution was extracted with EtOAc (30 mL×3). The organic phases were combined and washed with saturated salt solution, dried over anhydrous sodium sulfate and concentrated and evaporated to dryness, and purified by silica gel column chromatography (EtOAc: PE=1: 3) to give Compound 8c (1.00 g, 66.2% yield). ¹H NMR (400MHz, CDCl₃) δ: 3.80-3.73 (m, 1H), 3.62-3.57 (m, 2H), 3.21 (s, 3H), 2.37-2.30 (m, 2H), 2.08-2.02 (m, 1H), 1.67-1.60 (m, 2H).

Preparation of Compound 8d: Compound 8c (1.00 g, 8.61 mmol) was dissolved in 15 mL DCM, and the solution was added with TEA (2.61 g, 25.83 mmol) and dropwise added with MsCI (1.48 g, 12.92 mmol) slowly in an ice bath. After addition finished, the mixture was stirred for 2 h, and the reaction was monitored by TLC until the reaction finished. The mixture was added with 50 mL saturated NaHCO₃ solution and extracted with DCM (30 mL×3). The organic phases were combined and washed with saturated salt solution, dried over anhydrous sodium sulfate, concentrated and evaporated to dryness to be used.

Preparation of Compound 8e: Compound 10 (0.46 g, 1.29 mmol), Compound 8d (0.50 g, 2.58 mmol) and K₂CO₃ (0.53 g, 3.87 mmol) were added into 10mL anhydrous DMF, and the mixture was heated to 90 °C and stirred for 5 h. The reaction was monitored by TLC until the reaction finished. The mixture was poured into water and extracted with EtOAc (30 mL×3). The organic phases were combined and washed with saturated salt solution, dried over anhydrous sodium sulfate and concentrated and evaporated to dryness to give 0.50g Compound 8e crude product.

Preparation of Compound I-8: Compound 8e (0.50 g, 1.10 mmol) was dissolved in THF (15 mL), and the solution was added with 1N aq. NaOH (6.6 mL, 6.60 mmol) and the mixture was reacted at 35 °C for 3h. The reaction was monitored by TLC until the reaction finished. The mixture was added with 1N HCl to adjust pH = 1-2, and a solid precipitated. The solid was collected by filtration, and then recrystallized from diethyl ether and ethanol to give Compound I-8 product (270 mg, 57.3% yield). ¹H NMR (400MHz, CDCl₃) δ: 8.44 (s, 1H), 7.17 (s, 1H), 7.05 (s,1H), 6.72 (s, 1H), 4.07-4.05 (m, 2H), 3.91 (s, 3H), 3.88-3.82 (m, 2H), 3.36-3.31 (dd, *J*₁= 6 Hz, *J*₂ = 16.8 Hz, 1H), 3.25 (s, 3H), 3.08-3.03 (m, 1H), 2.53-2.48 (m, 2H), 2.42-2.38 (m, 1H), 1.83-1.77 (m, 3H), 0.94 (d, *J* = 6.4 Hz, 3H), 0.82 (d, *J* = 6.8 Hz, 3H); ESI-MS *m*/*z* 428.2 (M+H)⁺.

### Embodiment 13: Preparation of 6-isopropyl-10-methoxy-9-((3-methoxycyclobutyl)methoxy)-2-oxo-6,7-dihydro-2H-pyrido[2,1-α]isoquinoline-3-carboxylic acid (I-8-rac)

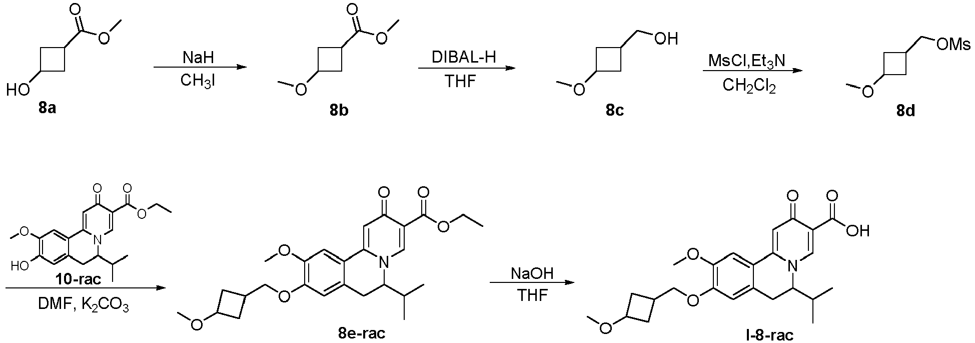

Compound I-8-rac was obtained according to the process of Embodiment 12. ¹H NMR (400MHz, CDCl₃) δ: 8.44 (s, 1H), 7.17 (s, 1H), 7.05 (s,1H), 6.72 (s, 1H), 4.07-4.05 (m, 2H), 3.91 (s, 3H), 3.88-3.82 (m, 2H), 3.36-3.31 (dd, *J*₁= 6 Hz, *J*₂ = 16.8 Hz, 1H), 3.25 (s, 3H), 3.08-3.03 (m, 1H), 2.53-2.48 (m, 2H), 2.42-2.38 (m, 1H), 1.83-1.77 (m, 3H), 0.94 (d, J = 6.4 Hz, 3H), 0.82 (d, *J* = 6.8 Hz, 3H); ESI-MS *m*/*z* 428.2 (M+H)⁺.

### Embodiment 14: Preparation of (S)-6-isopropyl-10-methoxy-2-oxo-9-((4-oxopentyl)oxy)-6,7-dihydro-2H-pyrido[2,1-α]isoquinoline-3-carboxylic acid (I-9)

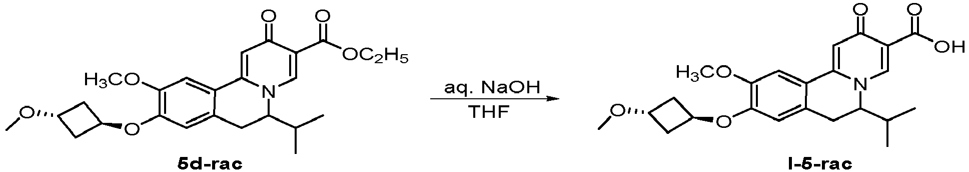

Preparation of Compound 9a: Compound 10 (0.36 g, 1.0 mmol) was dissolved in 10 mL THF, then 5-hydroxy-2-pentanone (0.20 g, 2.0 mmol) and PPh₃ (0.53 g, 2.0 mmol) were added. The mixture was replaced with nitrogen for four times and stirred in an ice-water bath for 30min. Then DEAD (0.35g, 2.0 mmol) was added into the above system by dropwise and the mixture was stirred at room temperature for 16 h. The mixture was mixed with silica gel and purified by column chromatography to give a product (0.25 g, 57.9% yield).

Preparation of Compound I-9: Compound 9a (0.25 g, 0.57 mmol) was dissolved in THF (10 mL) and water (5 mL), and the solution was added with 1N aq. NaOH (2.3 mL, 2.3 mmol) and then reacted at 35 °C for 3h. The reaction was monitored by TLC until the reaction finished. The mixture was adjusted to pH = 1-2 with 1N HCl and added with 20 mL DCM. The solution stood to layer, and the aqueous layer was extracted with DCM (30 mL×2). The organic layers were combined and washed with saturated salt solution, dried over anhydrous sodium sulfate, filtered and concentrated to give 300mg crude product. The crude product was dissolved in 2 mL ethanol, and the solution was refluxed for 20 min, then cooled to room temperature and 30 mL diethyl ether was added. The mixture was stirred for 30 min. The solution was suction filtered and dried to give Compound I-9 (130 mg, 55.1% yield). ¹H NMR (400MHz, CDCl₃) δ: 16.01 (s, 1H), 8.45 (s, 1H), 7.16 (s, 1H), 7.05 (s, 1H), 6.76 (s, 1H), 4.08-4.14 (m, 2H), 3.91 (s, 3H), 3.85-3.89 (m, 1H), 3.30-3.36 (m, 1H), 3.06 (d, *J* = 15.6Hz, 1H), 2.69 (t, *J* = 6.8Hz, 2H), 2.78 (s, 3H), 2.10-2.15 (m, 2H), 1.81 (m, 1H), 2.27 (m, 2H), 1.58(m, 2H), 0.94 (d, *J* = 6.4Hz, 3H), 0.81 (d, *J* = 6.4Hz, 3H); ESI-MS *m*/*z* 414.2 (M+H)⁺.

### Embodiment 15: Preparation of 6-isopropyl-10-methoxy-2-oxo-9-((4-oxopentyl)oxy)-6,7-dihydro-2H-pyrido[2,1-α]isoquinoline-3-carboxylic acid (I-9-rac)

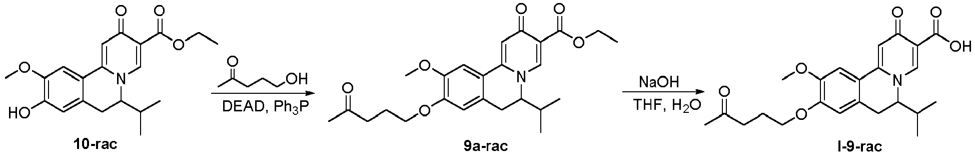

Compound I-9-rac was obtained according to the process of Embodiment 14. ¹H NMR (400MHz, CDCl₃) δ: 16.01 (s, 1H), 8.45 (s, 1H), 7.16 (s, 1H), 7.05 (s, 1H), 6.76 (s, 1H) , 4.08-4.14 (m, 2H), 3.91 (s, 3H), 3.85-3.89 (m, 1H), 3.30-3.36 (m, 1H), 3.06 (d, *J* = 15.6Hz, 1H), 2.69 (t, *J* = 6.8Hz, 2H), 2.78 (s, 3H), 2.10-2.15 (m, 2H), 1.81 (m, 1H), 2.27 (m, 2H), 1.58 (m, 2H), 0.94 (d, *J* = 6.4Hz, 3H), 0.81 (d, *J* = 6.4Hz, 3H); ESI-MS *m*/*z* 414.2 (M+H)⁺.

### Embodiment 16: Preparation of (S)-9-(cyclopentyloxy)-6-isopropyl-10-methoxy-2-oxo-6,7-dihydro-2H-pyrido[2,1-α]isoquinoline-3-carboxylic acid (I-10)

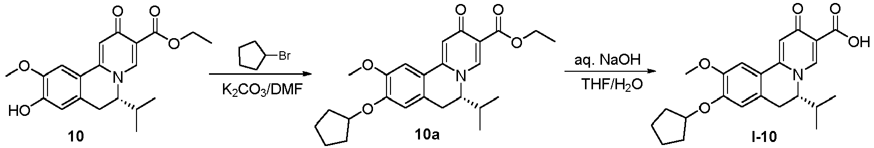

Preparation of Compound 10a: Compound 10 (180 mg, 0.50 mmol) was dissolved in 20 mL DMF, and the solution was added with bromocyclopentane (100 mg, 1.5 mmol) and K₂CO₃ (138 mg, 1.0 mmol) and reacted at 85°C for 3 h. The mixture was cooled to room temperature and added with 60 mL water and 50 mL EtOAc. The mixture stood to layer, and the aqueous layer was extracted with EtOAc (30 mL×3). The organic layers were combined and washed with saturated salt solution for once, and concentrated to give 300mg Compound 10a crude product, which was directly used in the next step.

Preparation of Compound I-10: Compound 10a (300 mg, 0.71 mmol) was dissolved in THF (10 mL) and water (5 mL), and the solution was added with 1N aq. NaOH (2.8 mL, 2.8 mmol) and stirred at room temperature for 16h. The reaction was monitored by TLC until the reaction finished. The mixture was added with 1N HCl to adjust pH=1-2 and added with 20 mL DCM. The mixture was separated and the aqueous layer was extracted with DCM (30 mL×2). The organic layers were combined and washed with saturated salt solution, dried over anhydrous sodium sulfate, filtered and concentrated to give 300 mg crude product. The crude product was dissolved in 2 mL ethanol and refluxed for 20 min, then cooled to room temperature and 30 mL diethyl ether was added. The mixture was stirred for 30 min. The solution was suction filtered and dried to give Compound I-10 (110 mg, 39.4% yield). ¹H NMR (400MHz, CDCl₃) δ: 8.44 (s, 1H), 7.15 (s, 1H), 7.04 (s, 1H), 6.72 (s, 1H), 4.82-4.87 (m, 1H), 3.90 (s, 3H), 3.84-3.89 (m, 1H), 3.31-3.36 (m, 1H), 3.03-3.07 (d, *J* = 16Hz, 1H), 1.81-2.00 (m, 6H), 1.61-1.67 (m, 4H), 0.94 (d, *J* = 6.8Hz, 3H), 0.82 (d, *J* = 6.8Hz, 3H); ESI-MS *m*/*z* 398.2 (M+H)⁺.

### Embodiment 17: Preparation of 9-(cyclopentyloxy)-6-isopropyl-10-methoxy-2-oxo-6,7-dihydro-2H-pyrido[2,1-α]isoquinoline-3-carboxylic acid (I-10-rac)

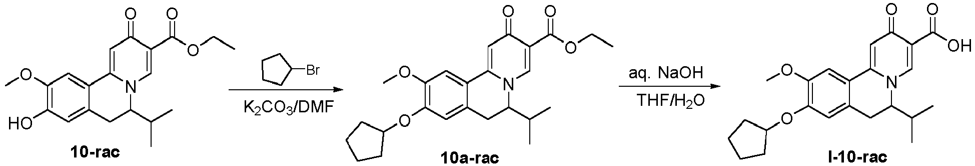

Compound I-10-rac was obtained according to the process of Embodiment 16. ¹HNMR (400MHz, CDCl₃) δ: 8.44 (s, 1H), 7.15 (s, 1H), 7.04 (s, 1H), 6.72(s, 1H), 4.82-4.87 (m,1H), 3.90 (s, 3H), 3.84-3.89 (m, 1H), 3.31-3.36 (m, 1H), 3.03-3.07 (d, *J* = 16Hz, 1H), 1.81-2.00 (m, 6H), 1.61-1.67 (m, 4H), 0.94 (d, *J* = 6.8Hz, 3H), 0.82 (d, *J* = 6.8Hz, 3H); ESI-MS *m*/*z* 398.2 (M+H)⁺.

### Embodiment 18: Preparation of (S)-9-(cyclopropylethynyl)-6-isopropyl-10-methoxy-2-oxo-6,7-dihydro-2H-pyrido[2,1-α]isoquinoline-3-carboxylic acid (I-11)

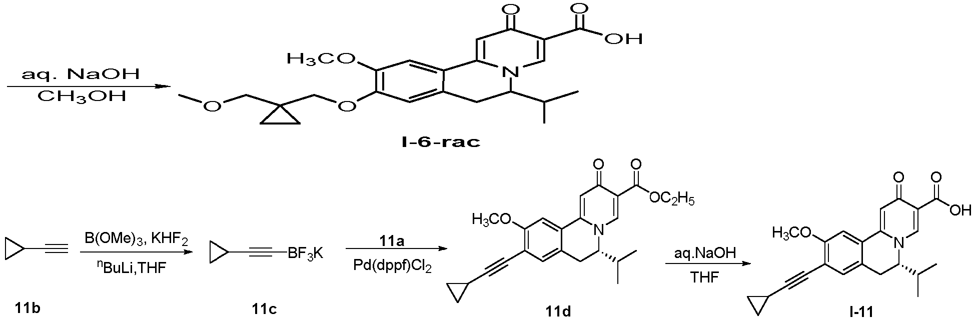

Preparation of Compound 11a: Compound 10 (0.50 g, 1.40 mmol) was dissolved in 10 mL DCM, and the solution was added with TEA (0.42 g, 4.2 mmol) and a solution of N-phenyl-bis(trifluoromethanesulfonimide) (0.75 g, 2.1 mmol) in DCM (6 mL) by dropwise for a period of about 3 min. The mixture was stirred for 30min at 0 °C and 2h at room temperature. After vacuum concentration, the crude product was purified by column chromatography to give Compound 11a (0.60 g, 87.6% yield).

Preparation of Compound 11c: Compound 11b (1.32 g, 20 mmol) was dissolved in 40 mL anhydrous THF, and the solution was replaced with nitrogen three times, cooled to - 78 °C and stirred at -78°C for 1h, then trimethyl borate (4.67 g, 45 mmol) was added. The solution was stirred at -78 °C for 1h, and then stirred at -20°C for 1h. A solution of saturated potassium hydrogen fluoride (14.06 g, 0.18 mol) was added at -20 °C, and the mixture was stirred vigorously for 1h and then at room temperature for 1h. The solvent was removed at reduced pressure to give a white solid. The white solid was vacuum dried and dissolved in 40mL hot acetone, filtered, and the filtrate was spin-dried and the residue was dissolved in 5 mL acetone, the solution was heated to reflux and then added with 50 mL diethyl ether, and the mixture was cooled to 0°C and filtered. The filter cake was vacuum dried to give Compound 11c (1.72 g, 50% yield).

Preparation of Compound 11d: Compound 11a (0.25 g, 0.51 mmol), Compound 11c (0.12 g, 0.66 mmol), Pd(dppf)Cl₂ (37 mg, 0.051 mmol) and sodium carbonate (0.11 g, 1.02 mmol) were added into a mixed solvent of TBME (12 mL) and water (3 mL), and the solution was replaced with nitrogen three times and heated to 100 °C and then reacted for 3h. The reaction was monitored by TLC until the reaction finished. The reaction liquid was pufiried by column chromatography to give Compound 11d (98 mg, 47.1% yield).

Preparation of Compound I-11: Compound 11d (98 mg, 0.24 mmol) was dissolved in THF (3 mL), and the solution was added with 1N aq. NaOH (1.3 mL, 1.3 mmol), and the mixture was reacted at room temperature for 18h. The mixture was added with 1N HCl to adjust pH = 1-2 and extracted with DCM (30 mL×2). The organic layers were combined and dried over anhydrous sodium sulfate, filtered and spin-dried to give a crude product. The crude product was recrystallized from EtOAc and TBME to give Compound I-11 (32 mg, 35.3% yield). ¹H NMR (400MHz, CDCl₃) δ: 8.46 (s, 1H), 7.25 (s, 1H), 7.16-7.11 (m, 2H), 3.93 (s, 3H), 3.88-3.82 (m, 1H), 3.51-3.44 (m, 1H), 3.30-3.21 (m, 1H), 3.10-3.03 (m, 1H), 1.57-1.49 (m, 1H), 1.29-1.23 (m, 1H), 0.96-0.78 (m, 10H); ESI-MS *m*/*z* 378.2 (M+H)⁺.

### Embodiment 19: Preparation of 9-(cyclopropylethynyl)-6-isopropyl-10-methoxy-2-oxo-6,7-dihydro-2H-pyrido[2,1-α]isoquinoline-3-carboxylic acid (I-11-rac)

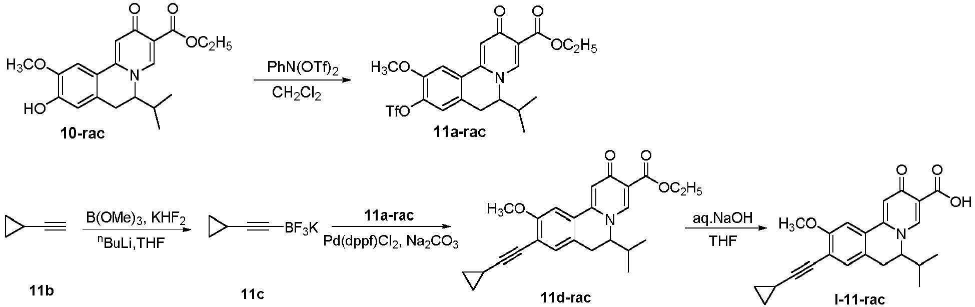

Compound I-11-rac was obtained according to the process of Embodiment 18. ¹H NMR (400MHz, CDCl₃) δ: 8.46 (s, 1H), 7.25 (s, 1H), 7.16-7.11 (m, 2H), 3.93 (s, 3H), 3.88-3.82 (m, 1H), 3.51-3.44 (m, 1H), 3.30-3.21 (m, 1H), 3.10-3.03 (m, 1H), 1.57-1.49 (m, 1H), 1.29-1.23 (m, 1H), 0.96-0.78 (m, 10H); ESI-MS *m*/*z* 378.2 (M+H)⁺.

### Embodiment 20: Preparation of (S)-6-isopropyl-10-methoxy-9-(4-methoxybut-1-yn-1-yl)-2-oxo-6,7-dihydro-2H-pyrido[2,1-α]isoquinoline-3-carboxylic acid (I-12)

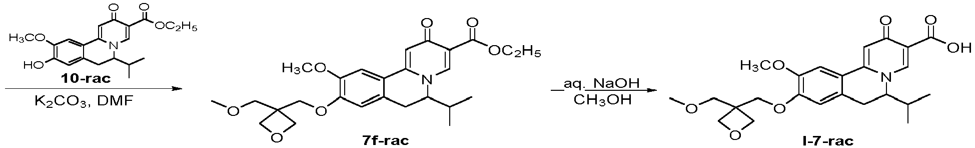

Preparation of Compound 12b: Compound 12a (1.30 g, 15.0 mmol) was dissolved in 30 mL anhydrous THF and, the solution was replaced with nitrogen three times, cooled down to -78 °C and stirred for 1h, then trimethyl borate (2.34 g, 22.0 mmol) was added, and the mixture was stirred at -78 °C for 1h, then stirred at -20°C for 1h. A saturated solution of potassium hydrogen fluoride (7.03 g, 90.0 mmol) was added and the mixture was stirred vigorously for 1h and then at room temperature for 1h. The solvent was removed at reduced pressure to give a white solid. The white solid was vacuum dried and dissolved in 40mL hot acetone, filtered, and the filtrate was spin-dried and the residue was dissolved in 5 mL acetone, the solution was heated to reflux and then added with 50 mL diethyl ether, and the mixture was cooled to 0 °C and filtered. The filter cake was vacuum dried to give a solid (0.32 g, 11.2% yield), i.e., Compound 12b.

Preparation of Compound 12c: Compound 11a (120 mg, 0.25 mmol), Compound 12b (71 mg, 0.37 mmol), Pd(dppf)Cl₂ (18 mg, 0.025 mmol) and sodium carbonate (78 mg, 0.74 mmol) were added into a mixed solvent of TBME (9 mL) and water (2 mL), and the solution was replaced with nitrogen three times and heated to 100°C and then reacted for 3h. The reaction was monitored by TLC until the reaction finished. The reaction liquid was pufiried by column chromatography to give Compound 12c (55 mg, 52.0% yield).

Preparation of Compound I-12: Compound 12c (55 mg, 0.13 mmol) in THF (3 mL) was added with 1N aq. NaOH (1.28 mL, 1.28 mmol) and the mixture was reacted at room temperature for 18h. The mixture was added with 1N HCl to adjust pH = 1-2 and extracted with DCM (30 mL×2). The organic layers were combined and dried over anhydrous sodium sulfate, filtered and spin-dried to give a crude product. The crude product was purified by preparative chromatography to give Compound I-12 (11 mg, 21.4% yield). ¹H NMR (400MHz, CDCl₃) δ: 8.49-8.48 (m, 1H), 7.33 (s, 1H), 7.18-7.17 (m, 2H), 3.96 (s, 3H), 3.92-3.85 (m, 1H), 3.64 (t, *J* = 7.2Hz, 2H), 3.44 (s, 3H), 3.32-3.25 (m, 1H), 3.14-3.07 (m, 1H), 2.80 (t, *J* = 6.4Hz, 2H), 0.95 (d, *J* = 6.4Hz, 3H), 0.91 (d, *J* = 6.4Hz, 3H); ESI-MS *m*/*z* 396.2 (M+H)⁺.

### Embodiment 21: Preparation of (S)-6-isopropyl-10-methoxy-9-((1s,3R)-3-methoxycyclobutoxy)-2-oxo-6,7-dihydro- 2H-pyrido[2,1-α]isoquinoline-3-carboxylic acid (I-19)

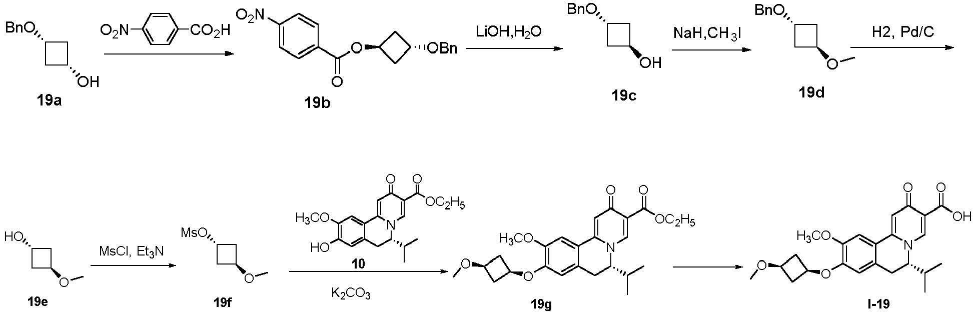

Preparation of Compound 19b: A mixture of compound 19a (1.60 g, 8.99 mmol), p-nitrobenzoic acid (1.50 g, 8.99 mmol) and triphenyl phosphine (4.24 g, 16.18 mmol) was dissolved in 10mL anhydrous THF, and the solution was replaced with nitrogen three times. The mixture was added with DEAD (2.35 g, 13.48 mmol) by dropwise in ice-water bath. The mixture was stirred at room temperature for 19 h and spin-dried to give a crude product, which was directly pufiried by column chromatography (PE to PE: EtOAc =50:1) to give 2.43 g solid (83.0% yield).

Preparation of Compound 19c: Compound 31b (2.43 g, 7.43 mmol) was dissolved in THF (3 mL) and methanol (1 mL), and the solution was added with a solution of LiOH (1.873 g, 44.6 mmol) in 10 mL H₂O. The mixture was stirred at room temperature for 4h. The organic solvent was removed by vacuum concentration, and the system was extracted with EtOAc (100ml×3). The EtOAc layers were combined and washed with water and saturated salt solution, dried over anhydrous sodium sulfate, filtered and vacuum concentrated to give 1.11g crude product.

Preparation of Compound 19d: Compound 3c (1.11 g, 6.21 mmol) was dissolved in THF (15 mL), and the solution was added with NaH (0.30 g, 12.42 mmol) and the mixture was stirred for 0.5h. Then Mel (1.15 g, 8.07 mmol) was added and the mixture was stirred at room temperature for 2 h after addition finished. The reaction was quenched by addition of 10mL water, and then the system was extracted with EtOAc (30ml×3). The EtOAc layers were combined, dried over anhydrous sodium sulfate and filtered, and the filtrate was directly mixed with silica gel and purified by column chromatography (PE: EA = 30: 1) to give a product (1.10 g, 92.1% yield).

Preparation of Compound 19e: Compound 31d (1.10 g, 5.72 mmol) was dissolved in 10 mL MeOH, and the solution was added with Pd/C (0.325 g) and 1 drop of concentrated HCl, and the mixture was replaced with hydrogen for three times and hydrogenated for 18h. The mixtre was filtered and the filtrate was vacuum concentrated to give a product (0.56 g, 95.9% yield).

Preparation of Compound 19f: Compound 31e (0.560 g, 5.49 mmol) dissolved in DCM (8 mL) was added with TEA (1.39 g, 13.72 mmol) and with MsCI (0.94 g, 8.23 mmol) slowly in ice-water bath. The mixture was stirred at room temperature for 2 h and spin-dried. The residue was added with 30 mL water and extracted with EtOAc (50 mL×2). The c organic phases were combined and washed with water and saturated salt solution, dried over anhydrous sodium sulfate, filtered and spin-dried to give 0.910g crude product.

Preparation of Compound 19g: Compound 10 (0.30 g, 0.84 mmol), Compound 19f (0.23 g, 1.26 mmol) were dissolved in 6mL DMF, and the solution was added with K₂CO₃ (0.35 g, 2.52 mmol) and reacted at 90°C for 18 h. After the reaction finished, the mixture was poured into water and extracted with EtOAc (50 mL×4). The EtOAc layers were combined and washed with water and saturated salt solution, dried over anhydrous sodium sulfate, filtered and vacuum concentrated to give 0.42g crude product.

Preparation of Compound I-19: Compound 19g (0.42 g, 0.95 mmol) was dissolved in THF (5 mL), and the solution was added with 10% aq. NaOH (0.30 g, 7.50 mmol) and the mixture was reacted for 18h. The mixture was added with 1N HCl to adjust pH = 1-2 and extracted with DCM (40 mL×2). The organic layers were combined and washed with saturated salt solution, dried and concentrated to give a crude product. The crude product was recrystallized from EA and TBME to give a product (0.170 g). ¹HNMR (400MHz, CDCl₃) δ: 8.47 (s,1H), 7.18 (s, 1H), 7.07 (s, 1H), 6.59 (s, 1H), 4.46-4.37 (m, 1H), 3.91-3.85 (m, 1H), 3.75-3.66 (m, 1H), 3.37-3.31 (m, 1H), 3.29 (s, 3H), 3.07 (d, *J* = 15.6 Hz, 1H), 2.98-2.91 (m, 2H), 2.32-2.20 (m, 2H), 1.87-1.79 (m, 1H), 0.95 (d, *J* = 6.4 Hz, 3H), 0.84 (d, *J* = 6.8 Hz, 3H). ESI-MS *m*/*z* 414.2 (M+H)⁺.

### Embodiment 22: Preparation of (S)-9-cyclobutoxy-6-isopropyl-10-methoxy-2-oxo-6,7-dihydro-2H-pyrido[2,1-α]isoquinoline-3-carboxylic acid (I-20)

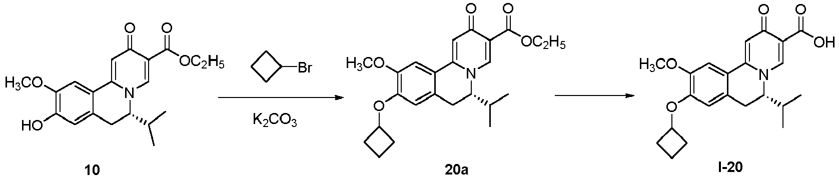

Preparation of Compound 20a: A mixture of Compound 10 (0.100 g, 0.28 mmol) and cyclobutylbromide (0.075 g, 0.56 mmol) was dissolved in 4 mL DMF, and the solution was added with K₂CO₃ (0.116 g, 0.84 mmol) and reacted at 90°C for 18 h. After the reaction finished, the mixture was poured into water and extracted with EtOAc (50 mL×4). The EtOAc layers were combined and washed with water and saturated salt solution, dried over anhydrous sodium sulfate and filtered, and the filtrate was vacuum concentrated to give 63 mg crude product. Yield: 54.6%.

Preparation of Compound I-20: Compound 20a (0.063 g, 0.15 mmol) was dissolved in THF (3 mL), and the solution was added with 10% aq. NaOH (0.200 g, 5 mmol) and the mixture was reacted at room temperature for 18h. The mixture was added with 1N HCl to adjust pH = 1-2 and extracted with DCM (40 mL×2). The organic layers were combined and washed with saturated salt solution, dried and concentrated to give a crude product. The crude product was recrystallized from EtOAc and TBME to give 15 mg product. Yield: 25.5%. ¹HNMR (400MHz, CDCl₃) δ: 8.50 (d, *J* = 0.8Hz, 1H), 7.16 (s, 1H), 7.06 (s, 1H), 6.57 (s, 1H), 4.77-4.67 (m, 1H), 4.64-4.48 (m, 2H), 3.92 (s, 3H), 3.41-3.26 (m, 1H), 3.09-2.98 (m, 1H), 2.56-2.44 (m, 2H), 2.37-3.20 (m, 2H), 1.97-1.85 (m, 2H), 1.75-1.68 (m, 1H), 0.93 (d, *J* = 6.4 Hz, 3H), 0.81 (d, *J* = 6.4 Hz, 3H). ESI-MS *m*/*z* 384.2 (M+H)⁺.

### Embodiment 23: Preparation of 10-chloro-6-isopropyl-2-oxo-9-(((R)-tetrahydrofuran-3-yl)oxy)-6,7-dihydro-2H-pyrido [2,1-α]isoquinoline-3-carboxylic acid (I-22-rac)

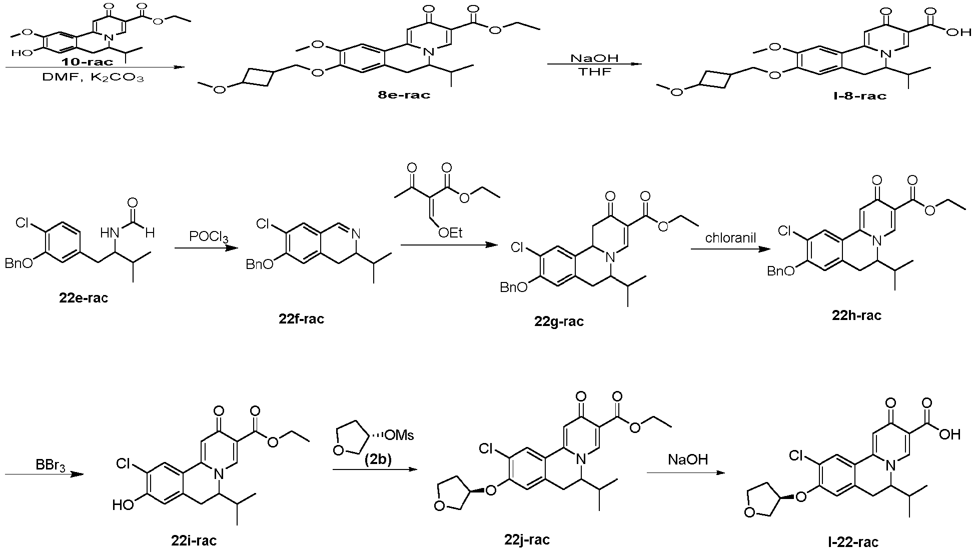

Preparation of Compound 22b: Compound 1 (2.07 g, 10 mmol) was dissolved in 20 mL ACN, and the solution was added with benzylbromide (2.05 g, 12 mmol) and K₂CO₃ (2.76 g, 20 mmol). The mixture was heated and stirred at 80 °C for 16 h. After the reaction finished, the mixture was added with 50 mL water and 50 mL EtOAc, and stood to layer. The aqueous layer was extracted with 50 mL EtOAc. The organic layer was washed with saturated salt solution, dried and concentrated, then purified by column chromatography to give 3.00 g product. Yield: 100%.

Preparation of Compound 22c: Compound 22b (3.00 g, 10 mmol) was dissolved in 50 mL THF. To this solution was added Pd₂(dba)₃ (185 mg, 0.2 mmol), Xantphos (234 mg, 0.4 mmol) and tBuONa (1.55 g, 16.2 mmol). The mixture was replaced with nitrogen three times. Then 4-methyl-2-butanone (1.74 g, 20.2 mmol) was added and the mixture was heated and stirred at 60 °C for 6h. The mixture was mixed with silica gel and then purified by column chromatography to give 2.70 g product. Yield: 89.4%.

Preparation of Compound 22d-rac: Compound 22c (2.70 g, 8.94 mol) was dissolved in 50 mL MeOH. To this solution was added ammonium acetate (6.90 g, 89.4 mmol) and NaBH₃CN (1.12 g, 17.88 mmol). The mixture was stirred at room temperature for 16 hours. The mixture was concentrated. The residue wasadded with aq. NaOH (1.40 g NaOH dissolved in 60 mL water) and DCM (50 mL), and the mixture was stirred for 20 min and stood to layer. The organic phase was separated and the aqueous layer was extracted with DCM (50 mL×2). The organic layers were combined and washed saturated salt solution, dried over anhydrous sodium sulfate and concentrated to give 2.70g crude product, which was directly used in the next step.

Preparation of Compound 22e-rac: Compound 22d-rac (2.70 g, 8.94 mmol) was dissolved in 30 mL dioxane. To this solution was added HCO₂H (3.50 g, 44.7 mmol) and the mixture was heated and refluxed for 3h. The mixture was cooled to room temperature and added with saturated NaHCO₃ (50 mL) and EtOAc (50 mL) and stood to layer. The organic layer was separated and the aqueous layer was extracted with EtOAc (50 mL×2). The organic layers were combined and washed with saturated salt solution, dried over anhydrous sodium sulfate and concentrated to give 3.20g crude product, which was directly used in the next step.

Preparation of Compound 22f-rac: Compound 22e-rac (3.20 g, 8.86 mmol) was dissolved in 50 mL acetonitrile. To this solution was added POCl₃ (1.63 g, 10.64 mmol) was added, and the mixture was stirred at room temperature for 16h. The reaction liquid was poured into 50 mL water and acetonitrile was removed by concentration. Then aqua ammonia was added to adjust pH = 8-9 and the mixture was added with 50 mL DCM (×2) and stood to layer. The organic phase was separated and the aqueous layer was extracted with DCM (50 mL×2). The organic layers were combined and washed with saturated salt solution, concentrated, mixed with silica gel and purified by column chromatography to give 1.80 g product. Yield: 64.8%.

Preparation of Compound 22g-rac: Compound 22f-rac (1.80 g, 5.74 mmol) was dissolved in 20 mL ethanol and 3 mL water. Then ethyl 2-ethoxymethylacetoacetate (3.20 g, 17.2 mmol) was added and the mixture was heated and stirred at 80 °C for 4h. The mixture was concentrated to remove ethanol and water to give 3.60g crude product, which was directly used in the next step.

Preparation of Compound 22h-rac: Compound 22g-rac (3.60 g, 7.96 mmol) was dissolved in DME (40 mL). To this solution was added tetrachlorobenzoquinone (1.96 g, 7.96 mmol) and the mixture was heated and stirred at 55 °C for 3h. The mixture was mixed with silica gel and purified by column chromatography to give 2.20 g product. Yield: 61.2%.

Preparation of Compound 22i-rac: Compound 22h-rac (2.10 g, 4.65 mmol) was dissolved in 30mL dried DCM and cooled in an ice bath. To this solution was added BBr₃ (2.33g, 9.29 mmol) slowly by dropwise and the mixture was cooled and stirred for 2 h. The mixture was poured into 50 mL ice water and extracted with DCM (50 mL×2). The organic layers were combined and washed with saturated NaHCO₃ and saturated salt solution, the residue was dried and concentrated to give 900 mg crude product, which was directly used in the next step.

Preparation of Compound 22j-rac: Compound 22i-rac (218 mg, 0.60 mmol) was dissolved in 20mL DMF, and the solution was added with an intermediate 2b (100 mg, 0.60 mmol) and K₂CO₃, and the mixture was heated and stirred at 85 °C for 3h. After cooling to room temperature, the mixture was added with 60 mL water and 50 mL EtOAc, and stood to layer. The organic phase was separated and the aqueous layer extracted with EtOAc (50 mL×2). The organic layers were combined and washed with saturated salt solution, dried and concentrated to give 300 mg crude product, which was directly used in the next step.

Preparation of Compound I-22-rac: To a solution of Compound 22j-rac (200 mg, 0.46 mmol) in THF (10 mL) and water (5 mL) was added NaOH (74 mg, 1.85 mmol) and the mixture was stirred at room temperature for 16h. The system was added with 1N HCl to adjust pH to 1-2 and added with 20mL DCM, and stood to layer. The organic phase was separated and the aqueous layer was extracted with DCM (30 mL×2). The organic layers were combined and washed with saturated salt solution for one time, dried and concentrated to give a crude product, which was purified by preparative chromatography to give 34 mg product. Yield: 18.3%. ¹H NMR (400 MHz, CDCl₃) δ: 8.47 (s, 1H), 7.77 (s, 1H), 7.04 (s, 1H), 6.74-6.76 (d, *J* = 6.0Hz, 1H), 5.05 (s, 1H), 3.91-4.10 (m, 4H), 3.34-3.40 (m, 1H), 3.11-3.15 (d, *J* = 15.6Hz, 1H), 2.21-2.31 (m,2H), 1.72-1.82 (m,2H), 1.25 (s, 1H), 0.94-0.95 (d, *J* = 6.4Hz, 3H), 0.82-0.84 (d, *J* = 6.8Hz, 3H). ESI-MS *m*/*z* 404.1 (M+H)⁺.

### Embodiment 24: Preparation of 10-chloro-6-isopropyl-9-((1R,3R)-3-methoxycyclobutoxy)-2-oxo-6,7-dihydro-2H-pyrido [2,1-α]isoquinoline-3-carboxylic acid (I-23-rac)

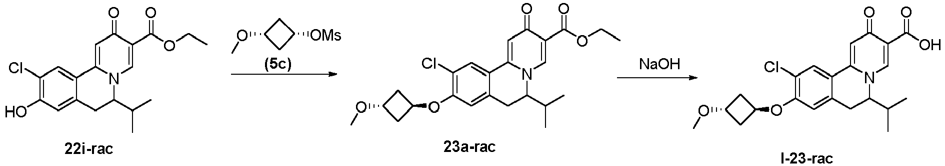

Preparation of Compound 23a-rac: Compound 22i-rac (80 mg, 0.25 mmol) was dissolved in 5mL DMF, and the solution was added with an intermediate 5c (90 mg, 0.50 mmol) and K₂CO₃ (166 mg, 1.20 mmol), and the mixture was heated and stirred at 85 °C for 3h. After cooling to room temperature, the mixture was added with 60 mL water and 50 mL EtOAc, and stood to layer. The organic phase was separated and the aqueous layer was extracted with EtOAc (50 mL×2). The organic layers were combined and washed with saturated salt solution, dried and concentrated to give 100 mg crude product, which was directly used in the next step.

Preparation of Compound I-23-rac: To a solution of Compound 23a-rac (100 mg, 0.22 mmol) in THF (10 mL) and water (5 mL) was added NaOH (40 mg, 1.00 mmol) and the mixture was stirred at room temperature for 16h. The system was added with 1N HCl to adjust pH = 1-2 and added with 20mL DCM, and stood to layer. The organic phase was separated and the aqueous layer was extracted with DCM (30 mL×2). The organic layers were combined and washed with saturated salt solution for one time, dried and concentrated, filtered and spin-dried to give a crude product, which is purified by preparative chromatography to give 17 mg product. Yield: 18.3%. ¹H NMR (400 MHz, CDCl₃) δ: 8.47 (s, 1H), 7.75(s, 1H), 7.04 (s, 1H), 6.89 (s, 1H), 4.95 (m, 1H), 4.15 (m, 1H), 3.90 (m, 1H), 3.33 (m, 4H, overlap), 3.13 (m, 1H), 2.50 (m, 4H), 1.77 (m, 1H), 0.95 (d, *J* = 6.8Hz, 3H), 0.84 (d, *J* = 6.8Hz, 3H); ESI-MS *m*/*z* 418.2 (M+H)⁺.

### Embodiment 25: Preparation of 10-chloro-6-isopropyl-9-((1-(methoxymethyl)cyclopropyl)methoxy)-2-oxo-6,7- dihydro-2H-pyrido[2,1-a]isoquinoline-3-carboxylic acid (I-24-rac)

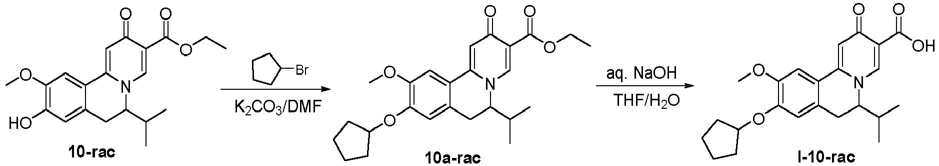

Preparation of Compound 24a-rac: Compound 22i-rac (80 mg, 0.25 mmol) was dissolved in 5mL DMF, and the solution was added with an intermediate 6c (90 mg, 0.50 mmol) and K₂CO₃ (138 mg, 1.00 mmol), and the mixture was heated and stirred at 85 °C for 3h. After cooling to room temperature, the mixture was added with 60 mL water and 50 mL EtOAc, and stood to layer. The organic phase was separated and the aqueous layer was extracted with EtOAc (50 mL×2). The organic layers were combined and washed with saturated salt solution, dried and concentrated to give 110 mg crude product, which was directly used in the next step.

Preparation of Compound I-24-rac: To a solution of Compound 24a-rac (110 mg, 0.24 mmol) in THF (10 mL) and water (5 mL) was added NaOH (40 mg, 1.00 mmol) and the mixture was stirred at room temperature for 16h. The system was added with 1N HCl to adjust pH to 1-2 and added with 20mL DCM, and stood to layer. The organic phase was separated and the aqueous layer was extracted with DCM (30 mL×2). The organic layers were combined and washed with saturated salt solution for one time, dried and concentrated to give a crude product, which was purified by preparative chromatography to give 23 mg product. Yield: 22.2%. ¹H NMR (400 MHz, CDCl₃) δ: 8.45 (s, 1H), 7.75(s, 1H), 7.03(s, 1H), 6.81 (s, 1H), 4.04 (m, 2H), 3.90 (m, 1H), 3.42 (m, 2H), 3.37 (s, 3H), 3.33 (m, 1H), 3.13-3.09 (m, 1H), 1.77 (m, 1H), 0.95 (d, *J* = 6.8 Hz, 3H), 0.82 (d, *J* = 6.4 Hz, 3H), 0.72 (m, 2H), 0.66 (m, 2H); ESI-MS *m*/*z* 432.1 (M+H)⁺.

### Embodiment 26: Preparation of 10-chloro-6-isopropyl-9-((3-(methoxymethyl)oxetan-3-yl)methoxy)-2-oxo-6,7- dihydro-2H-pyrido[2,1-α]isoquinoline-3-carboxylic acid (I-25-rac)

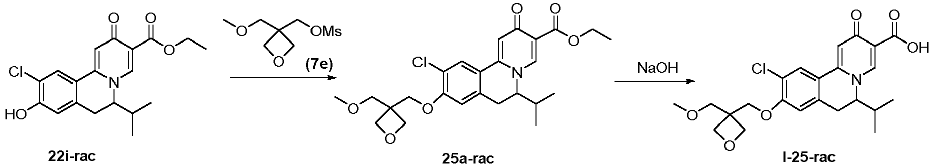

Preparation of Compound 25a-rac: Compound 22i-rac (80 mg, 0.25 mmol) was dissolved in 5mL DMF, and the solution was added with an intermediate 7e (105 mg, 0.50 mmol) and K₂CO₃ (138 mg, 1.00 mmol), and the mixture was heated and stirred at 85 °C for 3h. After cooling to room temperature, the mixture was added with 60 mL water and 50 mL EtOAc, and stood to layer. The organic phase was separated and the aqueous layer was extracted with EtOAc (50 mL×2). The organic layers were combined and washed with saturated salt solution, dried and concentrated to give 105 mg crude product, which was directly used in the next step.

Preparation of Compound I-25-rac: To a solution of Compound 25a-rac (105 mg, 0.22 mmol) in THF (10 mL) and water (5 mL) was added NaOH (40 mg, 1.00 mmol) and the mixture was stirred at room temperature for 16h. The system was added with 1N HCl to adjust pH to 1 to 2 and added with 20mL DCM, and stood to layer. The organic phase was separated and the aqueous layer was extracted with DCM (30 mL×2). The organic layers were combined and washed with saturated salt solution for one time, dried and concentrated to give a crude product, which was purified by preparative chromatography to give 21 mg product. Yield: 21.3%. ¹H NMR (400 MHz, CDCl₃) δ: 8.48 (s, 1H), 7.76(s, 1H), 7.04(s, 1H), 6.89(s, 1H), 4.62 (m, 2H), 4.55 (m, 2H), 4.30 (q, *J* = 8.8 Hz, 2H), 3.95 (m, 1H), 3.79 (m, 2H), 3.65 (s, 3H), 3.37 (m, 1H), 3.13 (m, 1H), 1.70 (m, 1H), 0.95 (d, *J* = 6.4Hz, 3H), 0.82 (d, *J* = 6.8Hz, 3H). ESI-MS *m*/*z* 448.1 (M+H)⁺.

### Embodiment 27: Preparation of 10-chloro-6-isopropyl-9-((3-methoxycyclobutyl)methoxy)-2-oxo-6,7-dihydro-2H-pyrido[2,1-α]isoquinoline-3-carboxylic acid (I-26-rac)

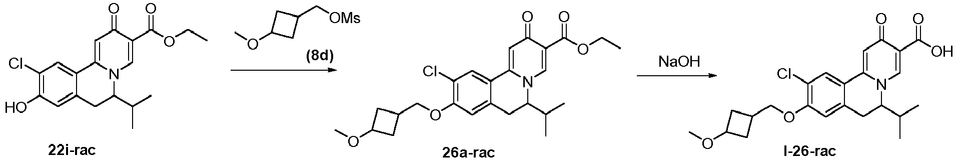

Preparation of Compound 26a-rac: Compound 22i-rac (186 mg, 0.52 mmol) was dissolved in 20mL DMF, and the solution was added with an intermediate 8d (100 mg, 0.52 mmol) and K₂CO₃ (142 mg, 1.04 mmol), and the mixture was heated and stirred at 85 °C for 3h. After cooling to room temperature, the mixture was added with 60 mL water and 50 mL EtOAc, and stood to layer. The organic phase was separated and the aqueous layer was extracted with EtOAc (50 mL×3). The organic layers were combined and washed with saturated salt solution once, dried and concentrated to give 200 mg crude product, which was directly used in the next step.

Preparation of Compound I-26-rac: To a solution of Compound 26a-rac (200 mg, 0.44 mmol) in THF (10 mL) and water (5 mL) was added NaOH (70 mg, 1.74 mmol) and the mixture was stirred at room temperature for 16h. THF was removed by concentration, and the system was added with 1N HCl to adjust pH to 1 to 2 and extracted with DCM (30 mL×3). The organic layers were combined and washed with saturated salt solution for one time, dried and concentrated to give a crude product, which was purified by preparative chromatography to give 12 mg product. Yield: 6.32%. ¹H NMR(400MHz, CDCl₃) δ: 8.47(s, 1H), 7.74(s, 1H), 7.02(s, 1H), 6.79(s, 1H), 4.06 - 4.10(m, 2H), 3.92 - 3.95(m, 1H), 3.83 - 3.87(m, 1H), 3.34 - 3.39(m, 1H), 3.25(s, 1H), 3.10 - 3.14(d, J= 16.4Hz, 1H), 2.37 - 2.52(m, 3H), 1.80 - 1.89(m, 2H), 1.21(s, 1H), 0.94 - 0.95(d, J= 6.4Hz, 3H), 0.81-0.83(d, *J*=6.8Hz, 3H). ESI-MS *m*/*z* 432.2(M+H)⁺.

### Embodiment 28: Preparation of 6-(tert-butyl)-10-methoxy-2-oxo-9-(((R)-tetrahydrofuran-3-yl)oxy)-6,7-dihydro-2H- pyrido[2,1-α]isoquinoline-3-carboxylic acid (I-28-rac)

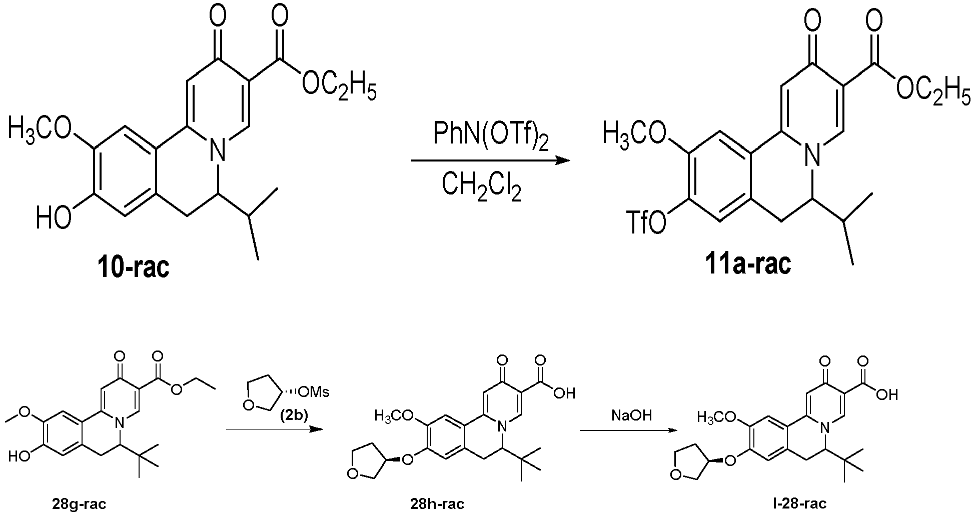

Preparation of Compound 28a: Compound 2 (4.40 g, 15 mmol) was dissolved in 50 mL THF. To this solution was added Pd₂(dba)₃ (275 mg, 0.3 mmol), Xantphos (347 mg, 0.6 mmol) and tBuONa (2.30 g, 24 mmol). The mixture was replaced with nitrogen three times. Then pinacolone (3.00 g, 15 mmol) was added and the mixture was heated and stirred at 60 °C for 6h. The mixture was mixed with silica gel and purified by column chromatography to give 3.30 g product. Yield: 70.7%.

Preparation of Compound 28b-rac: Compound 28a (3.30 g, 10.6 mmol) was dissolved in 50 mL MeOH. To this solution was added ammonium acetate (8.14 g, 0.11 mol) and NaBH₃CN (995 mg, 15.8 mmol). The mixture was stirred at room temperature for 16 hours. The mixture was concentrated to remove MeOH. The residue was added with aq. NaOH (1.40 g NaOH dissolved in 60 mL H₂O) and DCM (50 mL), and the mixture was stirred for 20 min and stood to layer. The organic phase was separated and the aqueous layer was extracted with DCM (50 mL×2). The organic layers were combined and washed saturated salt solution for one time, dried over anhydrous sodium sulfate and concentrated to give 3.40g crude product, which was directly used in the next step.

Preparation of Compound 28c-rac: Compound 28b-rac (3.40 g, 10.9 mmol) was dissolved in 50 mL dioxane. To this solution was added HCO₂H (2.51 g, 54.5 mmol) and the mixture was heated and refluxed for 3h. The mixture was cooled to room temperature and added with saturated NaHCO₃ (50 mL) and extracted with EtOAc (50 mL×3). The organic layers were combined and washed with saturated salt solution for one time, dried over anhydrous sodium sulfate and concentrated to give 3.40g crude product, which was directly used in the next step.

Preparation of Compound 28d-rac: Compound 28c-rac (3.40 g, 10 mmol) was dissolved in 50 mL acetonitrile. To this solution was added POCl₃ (1.85 g, 12 mmol) and the mixture was stirred at room temperature for 16h. The reaction liquid was poured into 50 mL water slowly and acetonitrile was removed by concentration. Then aqua ammonia was added to adjust pH to 8-9 and the mixture was extracted with DCM (50 mL×3). The organic layers were combined and washed with saturated salt solution, dried over anhydrous sodium sulfate and concentrated to give 3.20g crude product, which was directly used in the next step.

Preparation of Compound 28e-rac: Compound 28d-rac (3.20 g, 9.9 mmol) was dissolved in 40 mL ethanol and 5 mL water. Then ethyl 2-ethoxymethylacetoacetate (5.50 g, 29.7 mmol) was added and the mixture was heated and stirred at 80 °C for 4h. The mixture was concentrated to remove ethanol to give 6.40g crude product, which was directly used in the next step.

Preparation of Compound 28f-rac: Compound 28e-rac (6.40 g, 13.8 mmol) was dissolved in DME (40 mL). To this solution was added tetrachlorobenzoquinone (3.40 g, 13.8 mmol) and the mixture was heated and stirred at 55 °C for 3h. The mixture was mixed with silica gel and purified by column chromatography to give 3.20 g product. Yield: 50.2%.

Preparation of Compound 28g-rac: Compound 28f-rac (3.20 g, 6.9 mmol) was dissolved in MeOH (40 mL). To this solution was added 200mg Pd/C, and the mixture was replaced with hydrogen four times and stirred at room temperature for 16h. The mixture was suction filtered through diatomite, and the filtrate was concentrated to give 2.40g crude product.

Preparation of Compound 28h-rac: Compound 28g-rac (223 mg, 0.60 mmol) was dissolved in 20mL DMF, and the solution was added with an intermediate 2b (100 mg, 0.60 mmol) and K₂CO₃ (166 mg, 1.20 mmol), and the mixture was heated and stirred at 85 °C for 3h. After cooling to room temperature, the mixture was added with 60 mL water and 50 mL EtOAc, and stood to layer. The organic phase was separated and the aqueous layer was extracted with 50 mL EtOAc. The organic layer was washed with saturated salt solution for one time, and concentrated to give 300 mg crude product, which was directly used in the next step.

Preparation of Compound I-28-rac: To a solution of Compound 28h-rac (300 mg, 0.68 mmol) in THF (10 mL) and water (5 mL) was added NaOH (109 mg, 2.72 mmol) and the mixture was stirred at room temperature for 16h. The system was added with 1N HCl to adjust pH to 1 to 2 and extracted with DCM (30 mL×3). The organic layers were combined and washed with saturated salt solution for one time, dried and concentrated to give a crude product, which was purified by preparative chromatography to give 100 mg product. Yield: 35.5%. ¹H NMR (400 MHz, CDCl₃) δ: 8.47 (s, 1H), 7.16 (s, 1H), 7.07 (s, 1H), 6.65-6.66 (d, *J* = 4.8Hz, 1H), 5.04 (s, 1H), 4.01-4.06 (m, 4H), 3.91 (s, 3H), 3.39-3.45 (m, 1H), 3.14-3.18 (d, *J* = 16.8Hz, 1H), 2.21-2.26 (m, 2H), 1.25 (s, 1H), 0.81 (s, 9H). ESI-MS *m*/*z* 414.2 (M+H)⁺.

### Embodiment 29: Preparation of 6-(tert-butyl)-10-methoxy-9-((1R,3R)-3-methoxycyclobutoxy)-2-oxo-6,7-dihydro-2H- pyrido[2,1-α]isoquinoline-3-carboxylic acid (I-29-rac)

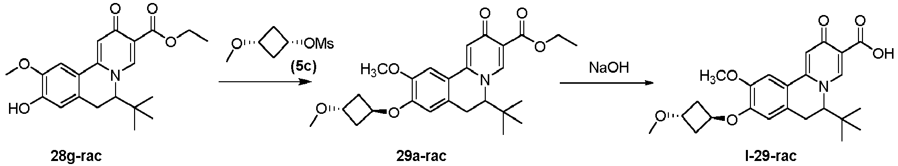

Preparation of Compound 29a-rac: Compound 28g-rac (65 mg, 0.18 mmol), Compound 5c (47 mg, 0.26 mmol) and K₂CO₃ (50 mg, 0.36 mmol) were added into 5mL DMF, and the mixture was replaced with nitrogen three times and then heated and stirred at 90° C for 3h. After the reaction finished, the mixture was diluted with water and extracted with EtOAc (20 mL×3). The organic phases were combined, dried over anhydrous sodium sulfate, and concentrated to give 75 mg crude product.

Preparation of Compound I-29-rac: Compound 29a-rac (75 mg, 0.16 mmol) was dissolved in THF (4 mL) and the solution was added with NaOH (45 mg) and water (1 mL), and the mixture was stirred at 50 °C for 3h. After the reaction finished, the mixture was added with 1N HCl to adjust pH to about 2-3 and extracted with DCM (20 mL×3). The organic phases were combined, dried over anhydrous sodium sulfate and concentrated, and purified by thin layer chromatography to give 10 mg product. Yield: 14.6%. ¹H-NMR (CDCl₃, 400 MHz) δ: 8.45(s, 1H), 7.14 (s, 1H), 7.05 (s, 1H), 6.51 (s, 1H), 4.94 (m, 1H), 4.16 (m, 1H), 4.01 (m, 1H), 3.92 (s, 3H), 3.37-3.43 (m, 1H), 3.29 (s, 3H), 3.16 (m, 1H), 2.48-2.56 (m, 3H), 0.82 (s, 9H); ESI-MS *m*/*z* 428.2 (M+H)⁺

### Embodiment 30: Preparation of 6-(tert-butyl)-10-methoxy-9-((1-(methoxymethyl)cyclopropyl)methoxy)-2-oxo-6,7- dihydro-2H-pyrido[2,1-α]isoquinoline-3-carboxylic acid (I-30-rac)

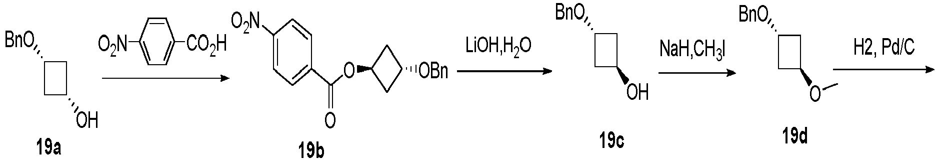

Preparation of Compound 30a-rac: Compound 28g-rac (85 mg, 0.23 mmol), Compound 6c (89 mg, 0.46 mmol) and K₂CO₃ (63 mg, 0.46 mmol) were added into 5mL DMF, and the mixture was replaced with nitrogen three times and then heated and stirred at 90 °C for 3h. After the reaction finished, the mixture was diluted with water and extracted with EtOAc (20 mL×3). The organic phases were combined, dried over anhydrous sodium sulfate, and concentrated to give 97 mg crude product.

Preparation of Compound I-30-rac: Compound 30a-rac (97 mg, 0.21 mmol) was dissolved in THF (4 mL) and the solution was added with NaOH (45 mg) and water (1 mL), and the mixture was stirred at 50 °C for 3h. After the reaction finished, the mixture was added with 1N HCl to adjust pH to about 2-3 and extracted with DCM (20 mL×3). The organic phases were combined, dried over anhydrous sodium sulfate, concentrated and purified by thin layer chromatography to give 26 mg product. Yield: 28.1%. ¹H-NMR (CDCl₃, 400 MHz) δ: 8.45 (s, 1H), 7.13 (s, 1H), 7.05 (s, 1H), 6.74 (s, 1H), 4.00 (m, 3H), 3.91 (s, 3H), 3.41 (s, 2H), 3.36 (s, 3H), 3.15 (m, 1H), 2.92 (m, 1H), 0.82 (s, 9H), 0.69-0.65 (m, 4H); ESI-MS m/z 442.2 (M+H)⁺

### Embodiment 31: Preparation of 6-(tert-butyl)-10-methoxy-9-((3-(methoxymethyl)oxetan-3-yl)methoxy)-2-oxo-6,7- dihydro-2H-pyrido[2,1-α]isoquinoline-3-carboxylic acid (I-31-rac)

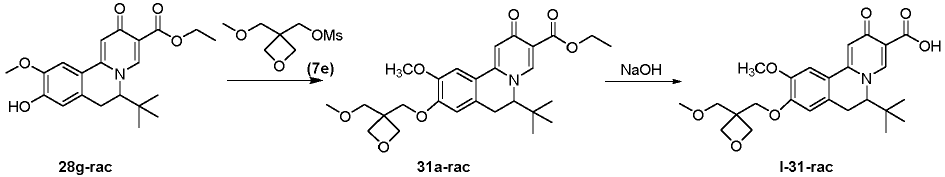

Preparation of Compound 31a-rac: Compound 28g-rac (65 mg, 0.18 mmol), Compound 7e (55 mg, 0.26 mmol) and K₂CO₃ (50 mg, 0.36 mmol) were added into 5mL DMF, and the mixture was replaced with nitrogen three times and then heated and stirred at 90 °C for 3h. After the reaction finished, the mixture was diluted with water and extracted with EtOAc (20 mL×3). The organic phases were combined, dried over anhydrous sodium sulfate and concentrated to give 87 mg crude product.

Preparation of Compound I-31-rac: Compound 31a-rac (87 mg, 0.18 mmol) was dissolved in THF (4 mL), and the solution was added with NaOH (45 mg) and water (1 mL), and the mixture was stirred at 50 °C for 3h. After the reaction finished, the mixture was added with 1N HCl to adjust pH to about 2-3 and extracted with DCM (20 mL×3). The organic phases were combined, dried over anhydrous sodium sulfate, concentrated and purified by thin layer chromatography to give 10 mg product. Yield: 12.1%. ¹H-NMR (CDCl₃, 400 MHz) δ: 8.46 (s, 1H), 7.15 (s, 1H), 7.07 (s, 1H), 6.79 (s, 1H), 4.61 (m, 4H), 4.27 (m, 2H), 4.01 (m, 1H), 3.89 (s,3H), 3.78 (m, 2H), 3.41 (s, 3H), 3.33 (m, 1H), 3.16 (m, 1H), 0.83 (s, 9H); ESI-MS *m*/*z* 458.2 (M+H)⁺.

### Embodiment 32: Preparation of 6-(tert-butyl)-10-methoxy-9-((3-methoxycyclobutyl)methoxy)-2-oxo-6,7-dihydro-2H- pyrido[2,1-α]isoquinoline-3-carboxylic acid (I-32-rac)

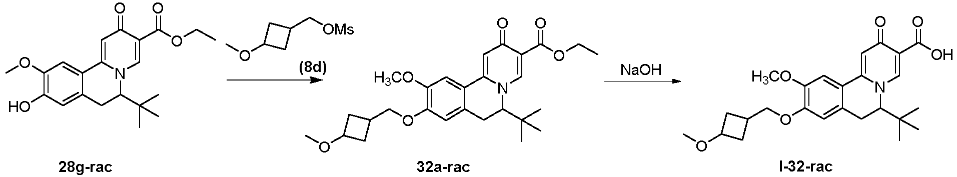

Preparation of Compound 32a-rac: Compound 28g-rac (191 mg, 0.52 mmol), Compound 8d (100 mg, 0.52 mmol) and K₂CO₃ (142 mg, 1.03 mmol) were added into 10mL DMF, and the mixture was replaced with nitrogen three times and then heated and stirred at 90 °C for 5h. After the reaction finished, the mixture was diluted with water and extracted with EtOAc (50 mL×3). The organic phases were combined, dried over anhydrous sodium sulfate, and concentrated to give 200 mg crude product.

Preparation of Compound I-32-rac: Compound 32a-rac (200 mg, 0.43 mmol) was dissolved in 10 mL THF, and the solution was added with NaOH (102 mg) and water (5 mL), and the mixture was stirred at 35 °C for 2h. After the reaction finished, the mixture was added with 1N HCl to adjust pH to about 2-3 and extracted with DCM (20 mL×3). The organic phases were combined, dried over anhydrous sodium sulfate, concentrated and purified by thin layer chromatography to give 70 mg product. Yield: 36.8%. ¹H-NMR (CDCl₃, 400 MHz) δ:8.48 (s, 1H), 7.15 (s, 1H), 7.07 (s, 1H), 6.71 (s, 1H), 4.03-4.08 (m, 3H), 3.92 (s, 3H), 3.40-3.46 (m, 1H), 3.15-3.19 (d, *J* = 15.2Hz, 1H), 2.22-2.55 (m, 3H), 1.78-1.82 (m, 2H), 1.26 (s, 1H), 0.82 (s, 9H). ESI-MS *m*/*z* 442.2 (M+H)⁺.

### Embodiment 33: Preparation of 10-methoxy-6-(1-methoxy-2-methylpropan-2-yl)-2-oxo-9-(((R)-tetrahydrofuran-3- yl)oxy)-6,7-dihydro-2H-pyrido[2,1-α]isoquinoline-3-carboxylic acid (I-52-rac)

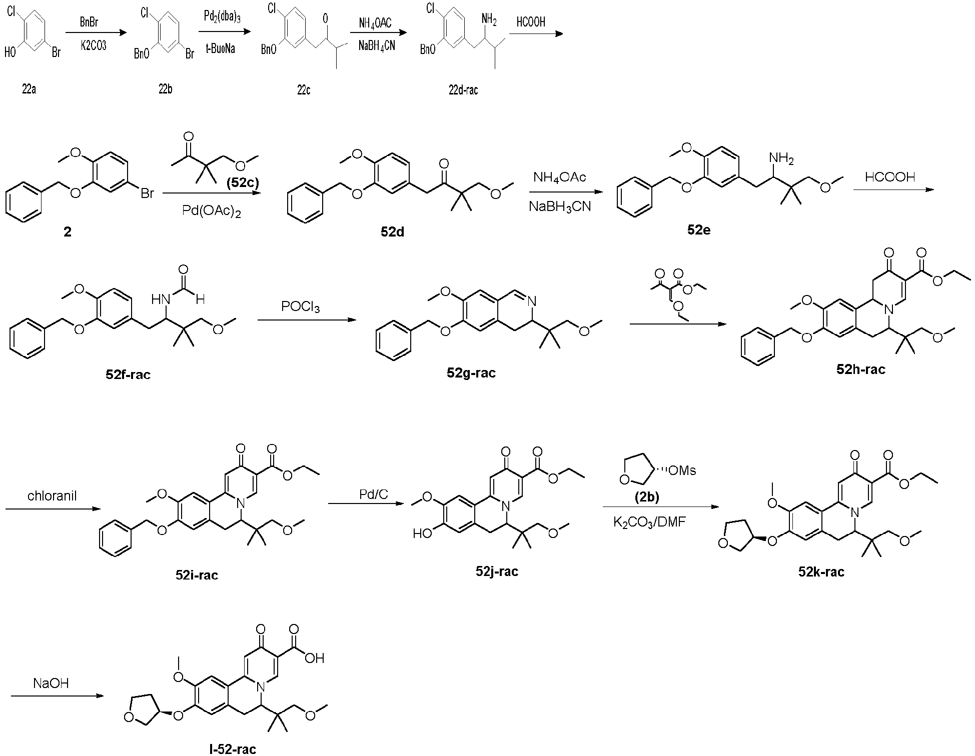

Preparation of Compound 52b: Compound 52a (10.0 g , 0.12 mol) was dissolved in 18 mL TFA, then 3.48 g paraformaldehyde was added and the mixture was stirred at 80 °C for 7h. After cooling, the mixture was added with 500 mL saturated NaHCO₃ and stirred for 6h. The mixture was extracted with DCM (100 mL×5). The organic phases were combined, dried over anhydrous sodium sulfate, concentrated and mixed with silica gel, and purified by column chromatography (PE: EtOAc =5:1) to give 5.0 g product . Yield: 35.8.

Preparation of Compound 52c: Compound 52b (5.0 g, 0.043mol) was added into 5.5 mL dimethyl sulfate, and the solution was added with 20N aq. NaOH (3mL). The mixture was stirred at 40 °C for 16h. The reaction was monitored by TLC until the reaction finished. The mixture was extracted with diethyl ether (30 mL×3). The organic phases were combined, dried over anhydrous sodium sulfate, and concentrated and evaporated to dryness to give 1.00g crude product.

Preparation of Compound 52d: Compound 2 (1.00 g, 3.41 mmol), Compound 52c (0.89 g, 6.82 mmol), Pd(OAc)₂ (12 mg , 1.5 %mol), Xphos (49 mg, 3 %mol) and 1M LiHMDS (10.2 mL, 10.2 mmol) were dissolved in 10mL dioxane, and the mixture was replaced with nitrogen three times. The mixture was heated and stirred at 70 °C for 3h. After the reaction finished, the reactive solvent was removed byspin-drying and the residue was purified by silica gel column chromatography with eluent PE: EtOAc = 5: 1 to give 0.62 g product. Yield: 53.4%.

Preparation of Compound 52e: Compound 52d (0.62 g, 1.82 mmol) was dissolved in 5 mL MeOH. To this solution was added ammonium acetate (1.40 g, 18.2 mmol) and the mixture was stirred for 30min. Then NaBH₃CN (0.23 g, 3.64 mmol) was added and the mixture was stirred at 60 °C for 16 hours. After the reaction finished, the mixture was added with 2N aq. NaOH (50 mL) and extracted with DCM (30 mL×3). The organic phases were combined, dried over anhydrous sodium sulfate, and concentrated and evaporated to dryness to give 0.63g crude product.

Preparation of Compound 52f-rac: Compound 52e (0.63 g, 1.84 mmol) was dissolved in 5 mL dioxane. To this solution was added 1 mL of HCO₂H and 0.5 mL of triethyl orthoformate and the mixture was heated and refluxed for 48h. After the reaction finished, the solvent was removed by spin-drying and the residue was extracted with DCM (20 mL×3). The organic phases were combined, dried over anhydrous sodium sulfate, concentrated and evaporated to dryness to give 0.60g crude product.

Preparation of Compound 52g-rac: Compound 52f-rac (0.60 g, 1.62 mmol) was dissolved in 5 mL acetonitrile. To this solution was added POCl₃ (0.30 mL, 3.24 mmol) in ice bath and the mixture was reacted at 60 °C in oil bath for 1h. After the reaction finished, the reactive solvent was removed by spin-drying and the residue was extracted with DCM (20 mL×3). The organic phases were combined, washed with saturated NaHCO₃, dried over anhydrous sodium sulfate and concentrated to give 0.58 g crude product.

Preparation of Compound 52h-rac: Compound 52g-rac (0.58 g, 1.64 mmol) and ethyl 2-ethoxymethylacetoacetate (0.92 g, 4.92 mmol) were dissolved in 6 mL ethanol, and the mixture was added with 2 mL water and heated, refluxed and stirred at reflux for 48h. After the reaction finished, the reactive solvent was removed by spin-drying to give 1.17g crude product.

Preparation of Compound 52i-rac: Compound 52h-rac (1.17 g, 2.36 mmol) and tetrachlorobenzoquinone (0.35 g, 1.42 mmol) were dissolved in DME (15 mL) and the mixture was heated, refluxed and stirred for 3h. After the reaction finished, the reactive solvent was removed by spin-drying and the residue was purified by silica gel column chromatography to give 475 mg product. Yield: 40.9%.

Preparation of Compound 52j-rac: Compound 52i-rac (475 mg, 0.97 mmol) was dissolved in MeOH (15 mL). To this solution was added 50mg Pd/C and the mixture was replaced with hydrogen three times and stirred at room temperature overnight under H₂ protection. The reaction was monitored by TLC until the reaction finished. After the reaction finished, the mixture was suction filtered, and the filtrate was evaporated to dryness to give 300 mg produc. Yield: 77.3%. ¹H-NMR (CDCl₃, 400 MHz): δ: 8.35 (d, 1H, *J* = 13.6 Hz), 7.11 (s, 1H), 6.91 (s, 1H), 6.80 (s, 1H), 4.37 (d, 2H, *J* = 6.4 Hz), 3.90 (s, 3H), 3.86 (s, 1H), 3.34 (s, 3H), 3.33-3.28 (m, 1H), 3.03 (s, 1H), 2.99-2.94 (m, 1H), 2.88-2.85 (m, 1H), 1.38-1.24 (m, 3H), 0.95 (d, 3H, *J* = 4Hz), 0.39 (d, 3H, *J* = 6.8 Hz).

Preparation of Compound 52k-rac: Compound 52j-rac (100 mg, 0.25 mmol), Compound 2b (83 mg, 0.50 mmol) and K₂CO₃ (103 mg, 0.75 mmol) were added in 5mL DMF and the mixture was replaced with nitrogen three times. The mixture was heated and stirred at 90 °C for 5h. After the reaction finished, the mixture was diluted with water and extracted with EtOAc (20 mL×3). The organic phases were combined, dried over anhydrous sodium sulfate, and purified by thin layer chromatography to give 75 mg product. Yield: 40.0%.

Preparation of Compound I-52-rac: Compound 52k-rac (75 mg, 0.1 mmol) was dissolved in THF (3 mL) and the solution was added with NaOH (32 mg) and water (1 mL), and the mixture was stirred at 35 °C for 2h. After the reaction finished, the mixture was added with 1N HCl to adjust pH to about 2-3 and extracted with DCM (20 mL×3). The organic phases were combined, dried over anhydrous sodium sulfate, concentrated and then purified by thin layer chromatography to give 30 mg product. Yield: 67.5%. ¹H-NMR (CDCl₃, 400 MHz) δ: 8.58 (s, 1H), 7.15 (s, 1H), 7.06 (s, 1H), 6.66 (s, 1H), 5.05-5.03 (m, 1H), 4.52-4.51 (m, 1H), 4.05-4.01 (m, 3H), 3.96-3.92 (m, 1H), 3.90 (s, 3H), 3.44-3.37 (m, 1H), 3.35 (s, 3H), 3.11-3.06 (m, 1H), 2.91 (s, 2H), 2.24-2.23 (m, 2H), 0.97 (s, 3H), 0.42 (s, 3H). ESI-MS *m*/*z* 444.2 (M+H)⁺.

### Embodiment 34: Preparation of 10-methoxy-6-(1-methoxy-2-methylpropan-2-yl)-9-((1R,3R)-3-methoxycyclobutoxy)-2-oxo-6,7-dihydro-2H-pyrido[2,1-α]isoquinoline-3-carboxylic acid (I-53-rac)

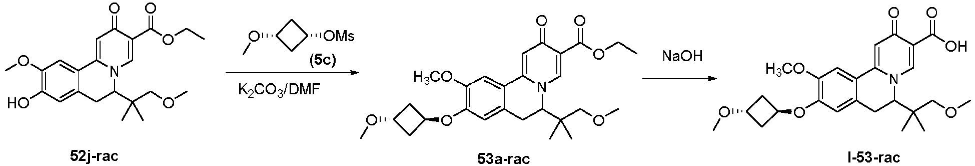

Preparation of Compound 53a-rac: Compound 52j-rac (100 mg, 0.25 mmol), 5c (90 mg, 0.50 mmol) and K₂CO₃ (103 mg, 0.75 mmol) were added in 5mL DMF and replaced with nitrogen three times. The mixture was heated and stirred at 90 °C for 5h. After the reaction finished, the mixture was diluted with water and extracted with EtOAc (20 mL×3). The organic phases were combined, dried over anhydrous sodium sulfate and concentrated to give 120 mg crude product.

Preparation of Compound I-53-rac: Compound 53a-rac (120 mg, 0.25 mmol) was dissolved in THF (3 mL), and the solution was added with NaOH (32 mg) and water (1 mL), and the mixture was stirred at 35 °C for 2h. After the reaction finished, the mixture was added with 1N HCl to adjust pH to about 2-3 and extracted with DCM (20 mL×3). The organic phases were combined, dried over anhydrous sodium sulfate, concentrated and purified by thin layer chromatography to give 20 mg product. Yield: 17.5%. ¹H-NMR (CDCl₃, 400 MHz) δ: 8.58 (s, 1H), 7.15 (s, 1H), 7.06 (s, 1H), 6.52 (s, 1H), 5.00-4.90 (m, 1H), 4.52-4.50 (m, 1H), 4.20 (m, 1H), 3.97 (m, 1H), 3.93 (s, 3H), 3.64 (m, 1H), 3.36 (s, 3H), 3.31 (s, 3H), 3.08 (m, 1H), 2.91 (m, 2H), 2.59 (m, 4H), 0.88 (s, 3H), 0.41 (s, 3H). ESI-MS *m*/*z* 458.2 (M+H)⁺.

### Embodiment 35: Preparation of 10-methoxy-6-(1-methoxy-2-methylpropan-2-yl)-9-((1-(methoxymethyl)cyclopropyl) methoxy)-2-oxo-6,7-dihydro-2H-pyrido[2,1-α]isoquinoline-3-carboxylic acid (I-54-rac)

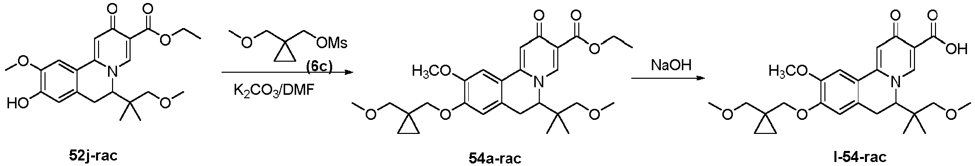

Preparation of Compound 54a-rac: Compound 52j-rac (100 mg, 0.25 mmol), Compound 6c (97 mg, 0.50 mmol) and K₂CO₃ (103 mg, 0.75 mmol) were added into 5mL DMF and the mixture was replaced with nitrogen three times. The mixture was heated and stirred at 90 °C for 5h. After the reaction finished, the mixture was diluted with water and extracted with EtOAc (20 mL×3). The organic phases were combined, dried over anhydrous sodium sulfate and concentrated to give 110 mg crude product.

Preparation of Compound I-54-rac: Compound 54a-rac (110 mg, 0.22 mmol) was dissolved in THF (3 mL), and the solution was added with NaOH (40 mg) and water (1 mL), and the mixture was stirred at 35 °C for 2h. After the reaction finished, the mixture was added with 1N HCl to adjust pH to about 2-3 and extracted with DCM (20 mL×3). The organic phases were combined, dried over anhydrous sodium sulfate, and concentrated and purified by thin layer chromatography to give 27 mg product. Yield: 26.1%. ¹H-NMR (CDCl₃, 400 MHz) δ:8.57 (s, 1H), 7.13 (s, 1H), 7.05 (s, 1H), 6.74 (s, 1H), 4.49 (d, *J* = 6.8Hz, 1H), 3.99 (s, 2H), 3.95 (d, *J* = 9.2 Hz, 1H), 3.90 (s, 3H), 3.41 (s, 2H), 3.36 (d, *J* = 4.8 Hz, 6H), 3.07 (d, *J* = 17.6 Hz, 1H), 2.91 (s, 2H), 0.97 (s, 3H), 0.71-0.63 (m, 4H), 0.43 (s, 3H); ESI-MS *m*/*z* 472.2 (M+H)⁺.

### Embodiment 36: Preparation of 10-methoxy-6-(1-methoxy-2-methylpropan-2-yl)-9-((3-(methoxymethyl)oxetan-3-yl) methoxy)-2-oxo-6,7-dihydro-2H-pyrido[2,1-α]isoquinoline-3-carboxylic acid (I-55-rac)

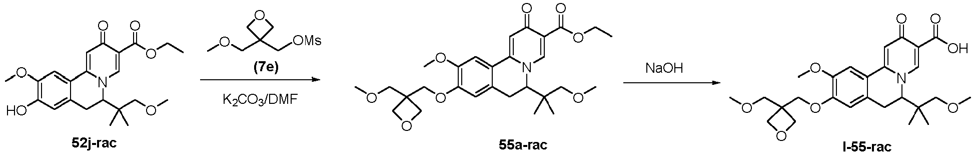

Preparation of Compound 55a-rac: Compound 67j-rac (54 mg, 0.13 mmol), Compound 7e (56 mg, 0.26 mmol) and K₂CO₃ (56 mg, 0.39 mmol) were added into 5mL DMF and the mixture was replaced with nitrogen three times. The mixture was heated and stirred at 90 °C for 5h. After the reaction finished, the mixture was diluted with water and extracted with EtOAc (20 mL×3). The organic phases were combined, dried over anhydrous sodium sulfate and concentrated to give 100 mg crude product.

Preparation of Compound I-55-rac: Compound 55a-rac (100 mg, 0.19 mmol) was dissolved in THF (4 mL), and the solution was added with NaOH (46 mg) and water (1 mL), and the mixture was stirred at 35 °C for 2h. After the reaction finished, the mixture was added with 1N HCl to adjust pH to about 2-3 and extracted with DCM (20 mL×3). The organic phases were combined, dried over anhydrous sodium sulfate, concentrated and purified by thin layer chromatography to give 10 mg product. Yield: 10.8%. ¹H-NMR (CDCl₃, 400 MHz) δ: 8.58 (s, 1H), 7.15 (s, 1H), 7.05 (s, 1H), 6.79 (s, 1H), 4.64-4.51 (m, 5H), 4.30-4.24 (m, 2H), 3.89 (s, 3H), 3.81-3.74 (m, 2H), 3.39 (d, 7H, *J* = 18.8 Hz), 3.12-3.08(m, 1H), 2.92 (s, 2H), 0.98 (s, 3H), 0.43 (s, 3H). ESI-MS *m*/*z* 488.2 (M+H)⁺.

### Embodiment 37: Preparation of 10-methoxy-6-(1-methoxy-2-methylpropan-2-yl)-9-((3-methoxycyclobutyl) methoxy)-2-oxo-6,7-dihydro-2H-pyrido[2,1-α]isoquinoline-3-carboxylic acid (I-56-rac)

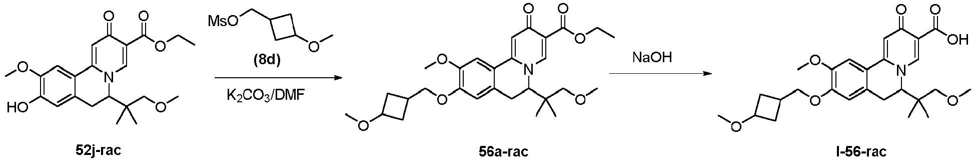

Preparation of Compound 56a-rac: Compound 52j-rac (100 mg, 0.25 mmol), Compound 8d (96 mg, 0.50 mmol) and K₂CO₃ (103 mg, 0.75 mmol) were added into 5mL DMF and the mixture was replaced with nitrogen three times. The mixture was heated and stirred at 90 °C for 5h. After the reaction finished, the mixture was diluted with water and extracted with EtOAc (20 mL×3). The organic phases were combined, dried over anhydrous sodium sulfate, concentrated and purified by thin layer chromatography to give 60 mg product. Yield: 40.0%.

Preparation of Compound I-56-rac: Compound 56a-rac (60 mg, 0.1 mmol) was dissolved in THF (4 mL), and the solution was added with NaOH (25 mg) and water (1 mL), and the mixture was stirred at 35 °C for 2h. After the reaction finished, the mixture was added with 1N HCl to adjust pH to about 2-3 and extracted with DCM (20 mL×3). The organic phases were combined, dried over anhydrous sodium sulfate, concentrated and purified by thin layer chromatography to give 40 mg product. Yield: 84.7%. ¹H-NMR(CDCl3, 400MHz) δ: 8.57 (s, 1H), 7.13(s, 1H), 7.04(s, 1H), 6.70(s, 1H), 4.51-4.50(m, 1H), 4.07-4.05(m, 2H), 3.90(s, 3H), 3.84(t, J=7.2Hz, 1H), 3.36(s, 3H), 3.25(s, 3H), 3.06(m, 1H), 2.91(s, 2H), 2.53-2.48(m, 1H), 2.42-2.38(m, 1H), 2.25-2.21(m, 1H), 1.82-1.77(m, 2H), 0.98(s, 3H), 0.42(s, 3H) ; ESI-MS *m*/*z* 472.2(M+H)⁺.

### Embodiment 38: In vitro bioactivity study

### Tested compounds:

The compounds of the present disclosure: Compound I-2, Compound I-5, Compound I-6, Compound I-7, Compound I-8, Compound I-9, Compound I-10, Compound I-11, Compound I-12, Compound I-19, Compound I-20, Compound I-1-rac, Compound I-2-rac, Compound I-3-rac, Compound I-4-rac, Compound I-5-rac, Compound I-6-rac, Compound I-7-rac, Compound I-8-rac, Compound I-9-rac, Compound I-10-rac, Compound I-11-rac, Compound I-22-rac, Compound I-23-rac, Compound I-24-rac, Compound I-25-rac, Compound I-26-rac, Compound I-28-rac, Compound I-29-rac, Compound I-30-rac, Compound I-31-rac, Compound I-32-rac, Compound I-52-rac, Compound I-53-rac, Compound I-54-rac, Compound I-55-rac, Compound I-56-rac;
The reference compounds: A, A-rac (the structures thereof are as follows):

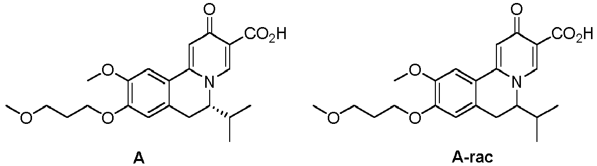

Test method: HepG2.2.15 cell line was seeded into 96-well plates at 1.5×10⁴ cells/well. On the following day, the cells were treated with a three-fold serial dilution series of the compounds at eight concentration points, and 2 duplicate wells were determined in parallel. The final DMSO concentration in the culture solution was 0.5%. On the fifth day, the culture solution was replaced with fresh solution containing the compounds. On the eighth day, the culture supernatant was collected and the HBsAg in the culture supernatant was detected by ELISA. The percent inhibition was calculated with respect to the blank control. See Table 1 for the results.

**Table 1: Activity in HBsAg inhibition of the compounds**

| Compound | EC₅₀ (nM) | Compound | EC₅₀ (nM) | Compound | EC₅₀ (nM) | Compound | EC₅₀ (nM) |
|---|---|---|---|---|---|---|---|
| I-2 | 1.82 | I-1-rac | 5.74 | I-22-rac | 5.46 | I-53-rac | 6.65 |
| I-5 | 1.61 | I-2-rac | 3.26 | I-23-rac | 6.83 | I-54-rac | 5.34 |
| I-6 | 3.15 | I-3-rac | 7.04 | I-24-rac | 5.78 | I-55-rac | 7.23 |
| I-7 | 3.82 | I-4-rac | 3.62 | I-25-rac | 6.97 | I-56-rac | 5.95 |
| I-8 | 1.54 | I-5-rac | 3.56 | I-26-rac | 7.56 | A | 4.06 |
| I-9 | 3.26 | I-6-rac | 4.12 | I-28-rac | 5.78 | A-rac | 8.21 |
| I-10 | 3.01 | I-7-rac | 4.68 | I-29-rac | 6.45 | | |
| I-11 | 2.57 | I-8-rac | 5.59 | I-30-rac | 6.74 | | |
| I-12 | 3.83 | I-9-rac | 3.71 | I-31-rac | 5.98 | | |
| I-19 | 2.89 | I-10-rac | 3.78 | I-32-rac | 7.31 | | |
| I-20 | 2.56 | I-11-rac | 2.61 | I-52-rac | 7.57 | | |

Conclusion: The compounds of the present disclosure have good ability to inhibit HBsAg, with activity below 10 nM.

### Embodiment 39: Rat PK study

Preparation of intravenous injection formulation: accurately weighing 2∼3mg of a sample, adding appropriate amount of N,N-dimethylacetamide (DMA), vortex oscillating to completely dissolve the solid matter; adding an appropriate volume of 30% Solutol HS-15 aqueous solution, vortex oscillating and then adding saline, so that DMA: 30% Solutol HS-15:saline = 20: 20: 60 (v/v/v), vortex oscillating the liquid to mix the liquid evenly, and filtering to obtain a pharmaceutical preparation of aconcentration of 0.4 mg·mL⁻¹.

Preparation of oral formulation: accurately weighing 10mg of a sample, adding an appropriate volume of 0.5% CMC-Na aqueous solution, vortex oscillating and ultrasounding to mix the liquid evenly to obtain a pharmaceutical preparation of aconcentration of 1 mg·mL⁻¹.

The formulations were freshly prepared on the day of administration, and samples were reserved and taken for the determination of the actual concentration.

S-D rats of Group A were given a single intravenous injection (IV) of 2 mg·kg⁻¹, respectively; S-D rats of Group B were given a single gavage administration (PO) of 10 mg·kg⁻¹, respectively. Approximately 0.15 mL of blood sample will was collected via jugular vein into EDTA-K2 tubes at the designated time points of pre-dose, and 5 min (IV only), 15 min, 30 min, 1 h, 2 h, 4 h, 8 h, 12 h and 24 h post dose. All of the whole blood samples were centrifuged for 10 min (at 5500 rpm) to obtain plasma samples, which were stored at a refrigerator below -30 ∼ -10°C. Concentrations of compounds in the plasma samples were analyzed using a LC-MS/MS method. The pharmacokinetic parameters were calculated using non-compartment model in Pharsight Phoenix 7.0 software. See Tables 2a and 2b for the results.

**Table 2a: PK parameters (IV) of tested compounds**

| PK (IV) | Compounds | | | | | | | | |
|---|---|---|---|---|---|---|---|---|---|
| | I-2 | I-5 | I-6 | I-7 | I-8 | I-9 | I-10 | I-11 | A |
| T_{1/2} (h) | 1.8 | 0.98 | 0.71 | 0.67 | 0.89 | 0.29 | 0.88 | 0.58 | 0.94 |
| AUC₀₋ₜ (ng·h·mL⁻¹) | 1797 | 2937 | 1016 | 1179 | 2147 | 504 | 1487 | 946 | 1607 |
| CL (mL·kg⁻¹·min⁻¹) | 18.6 | 11.3 | 33.6 | 28.8 | 15.7 | 65.9 | 22.7 | 35.9 | 20.8 |
| Vdₛₛ (L·kg⁻¹) | 1.51 | 0.858 | 1.74 | 1.39 | 1.05 | 0.942 | 1.41 | 1.37 | 1.17 |

**Table 2b: PK parameters (PO) of tested compounds**

| PK (PO) | Compounds | | | | | | | | |
|---|---|---|---|---|---|---|---|---|---|
| | I-2 | I-5 | I-6 | I-7 | I-8 | I-9 | I-10 | I-11 | A |
| T_{1/2} (h) | 1.6 | 1.7 | 1.9 | 1.4 | 1.3 | 1.8 | 1.3 | 3.2 | 1.5 |
| Tₘₐₓ (h) | 1.2 | 0.5 | 0.58 | 1 | 0.92 | 0.33 | 1 | 1.8 | 2.2 |
| Cₘₐₓ (ng·mL⁻¹) | 437 | 1343 | 1493 | 103 | 739 | 79.9 | 265 | 119 | 475 |
| AUC₀₋ₜ (ng·h·mL⁻¹) | 1687 | 5500 | 2310 | 470 | 3027 | 105 | 869 | 461 | 1763 |
| F (%) | 18.9 | 37.7 | 45.8 | 8.1 | 27.1 | 4.3 | 11.7 | 11.5 | 21.5 |

Conclusion: Combining with PK data of IV and PO, the compounds (especially Compounds I-2, I-5, I-6 and I-8) of the present disclosure have good PK properties and show good development prospects. It is probably because the cyclizations of the side chains in the chemical structures improve the intrinsic metabolic stabilities of the compounds.

The above description of the embodiments is only to assist in understanding the process of the present disclosure and its core concept. It should be noted that those skilled in the art can make various modifications and changes to the present disclosure without departing from the principles of the disclosure, and these modifications and changes are also intended to fall into the scope of the appended claims.

## Claims

1. An isoquinolinone compound of Formula (I) or a stereoisomer, pharmaceutically acceptable salt, solvate or crystal thereof: 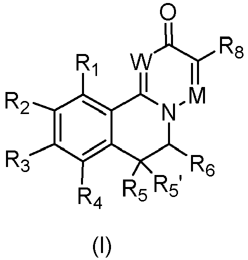 wherein:
(1) R₁ is selected from H, deuterium, C₁₋₆ alkyl, cyano, halogen, carboxyl, ester, C₃₋₆ cycloalkyl, C₄₋₈ heterocycloalkyl halogenated C₁₋₆ alkyl or C₆₋₁₀ aryl;
(2) R₂ is selected from halogen, C₁₋₃ alkoxy, deuterated C₁₋₃ alkoxy, C₁₋₆ alkyl, C₃₋₆ cycloalkyl, C₃₋₆ cycloalkyloxy, C₄₋₈ heterocycloalkyl C₁₋₆ alkyl, halogenated C₁₋₃ alkyloxy, halogenated C₃₋₆ cycloalkyl and C₃₋₆ cycloalkyl C₁₋₆ alkyl, or R₂ and R₃ are bonded by a carbon atom to form a ring;
(3) R₃ is (a) C₄₋₁₂ hydrocarbyl with a ring structure and/or an unsaturated bond, hydrogen in said C₄₋₁₂ hydrocarbyl is unsubstitued or substituted by one or more of deutrium, halogen, cyano, hydroxyl and sulphydryl, and said C₄₋₁₂ hydrocarbyl is uninterrupted by heteroatom or interrupted by one or more of O, S, NH, C=O, C=S, O=S=O, the heteroatom is selected from oxygen, sulphur or nitrogen; or (b) R₂ and R₃ are bonded by a carbon atom to form a ring;
(4) R₄ is selected from hydrogen, deuterium, halogen, cyano, ester or C₁₋₃ alkyl;
(5) R₅ and R₅' are independently selected from hydrogen, deuterium, halogen, methyl and methoxy, or R₅ and R₅' form a carbocyclic ring or a heterocyclic ring; or R₅ and R₆ form a carbocyclic ring or a heterocyclic ring;
(6) M is CH or N;
(7) R₆ is selected from C₁₋₆ alkyl, C₁₋₆ alkoxy C₁₋₆ alkyl, hydroxyl C₁₋₆ alkyl, aryl, halogenated C₁₋₆ alkyl, or C₃₋₆ cycloalkyl C₁₋₆ alkyl;
(8) W is N or CR₇, wherein R₇ is selected from hydrogen, deuterium, hydroxyl, halogen, C₁₋₃ alkyl, C₁₋₆ alkoxy, C₃₋₆ cycloalkyloxy, ester, carboxyl or cyano;
(9) R₈ is selected from carboxyl, ester, C₁₋₆ alkyl, C₃₋₆ cycloalkyl, C₁₋₆ alkyl alkynyl or C₃₋₆ cycloalkyl alkynyl; wherein, the alkyl portion of said ester is selected from C₁₋₆ alkyl, C₃₋₈ cycloalkyl, C₃₋₈ cycloalkyl alkynyl, C₁₋₆ alkyl alkynyl, benzyl, C₁₋₆ alkyl-C(O)O-C₁₋₃ alkyl and C₁₋₆ alkyl-OC(O)O-C₁₋₃ alkyl.

2. The isoquinolinone compound or the stereoisomer, the pharmaceutically acceptable salt, the solvate or the crystal thereof according to claim 1, is **characterized in that**, in said (a), the ring structure is a 3- to 8- membered ring; and, the unsaturated bond is a double bond or a triple bond.

3. The isoquinolinone compound or the stereoisomer, the pharmaceutically acceptable salt, the solvate or the crystal thereof according to claim 1, is **characterized in that**, in said (a), the ring structure is a saturated ring.

4. The isoquinolinone compound or the stereoisomer, the pharmaceutically acceptable salt, the solvate or the crystal thereof according to claim 1, is **characterized in that**, in said (a), the numbers of the ring structure and the unsaturated bond are 1 to 2, respectively.

5. The isoquinolinone compound or the stereoisomer, the pharmaceutically acceptable salt, the solvate or the crystal thereof according to claim 1, is **characterized in that**, in said (a), there is a 3- to 8- membered saturated carbocyclic ring or a 3- to 8- membered saturated heterocyclic ring, and at least one heteroatom, or at least one double or triple bond.

6. The isoquinolinone compound or the stereoisomer, the pharmaceutically acceptable salt, the solvate or the crystal thereof according to any one of claims 1 to 5, is **characterized in that**, in said (a), at least two of said ring structure, said unsaturated bond and said heteroatom are simultaneously present.

7. The isoquinolinone compound or the stereoisomer, the pharmaceutically acceptable salt, the solvate or the crystal thereof according to claim 6, is **characterized in that**, said (a) is a group satisfying any one of the conditions described in the following:
a1) having and only having one said ring structure and one unsaturated carbon-carbon bond;
a2) having both said ring structure and 1 - 3 heteroatoms, and at least one of the heteroatoms is oxygen, which is connected to a benzene ring in said Formula (I) through a single bond;
a3) having both said unsaturated bond and 1 - 3 heteroatoms, wherein the unsaturated bond is a carbon-carbon doulbe bond, a carbon-carbon triple bond or a carbon-oxygen doulbe bond, and when the unsaturated bond is a carbon-carbon doulbe bond or a carbon-carbon triple bond, one end thereof is preferably connected to the benzene ring in said Formula (I) through a single bond.

8. The isoquinolinone compound or the stereoisomer, the pharmaceutically acceptable salt, the solvate or the crystal thereof according to claim 1, is **characterized in that**, R₃ is selected from C₅₋₁₁ bicycloalkyl, C₃₋₆ cycloalkyl alkynyl, C₃₋₆ cycloalkyl alkenyl, C₁₋₃ alkoxy C₁₋₆ alkyl alkynyl, C₁₋₃ alkoxy C₁₋₆ alkyl alkenyl and C₄₋₈ heterocycloalkyl; or,
R₃ is R_{A}-O-, R_{A} is selected from C₃₋₈ cycloalkyl; C₅₋₁₁ bicycloalkyl; deuterated C₁₋₆ alkyl; C₄₋₈ heterocycloalkyl; C₁₋₆ alkyl carbonyl C₁₋₆ alkyl; deuterated C₁₋₃ alkoxyC₁₋₆ alkyl; C₁₋₃ alkoxy C₃₋₈ cycloalkyl; C₁₋₃ alkoxy C₃₋₈ cycloalkyl C₁₋₆ alkyl; C₃₋₈ heterocycloalkyl ; C₁₋₃ alkoxy C₁₋₆ alkyl, wherein alkyl is substituted by C₃₋₈ cycloalkyl or C₄₋₈ heterocycloalkyl, and a heteroatom in heterocycloalkyl is selected from oxygen, sulphur or nitrogen, when R_{A} is C₁₋₃ alkoxy C₁₋₆ alkyl, R₅ and R₅' are independently selected from deuterium, fluorine, chlorine, hydroxyl and cyano, and W is N or CR₇, wherein R₇ is selected from deuterium, fluorine, chlorine, hydroxyl, and cyano.

9. The isoquinolinone compound or the stereoisomer, the pharmaceutically acceptable salt, the solvate or the crystal thereof according to claim 1, is **characterized in that**, R₃ is selected from C₃₋₈ cycloalkoxy, C₃₋₈ heterocycloalkyloxy, C₁₋₃ alkoxy C₃₋₈ cycloalkoxy, C₁₋₃ alkoxy C₃₋₈ cycloalkyl C₁₋₆ alkoxy, C₃₋₈ heterocycloalkyl, C₁₋₃ alkoxy C₂₋₉ alkenyl, C₁₋₃ alkoxy C₂₋₉ alkynyl, C₃₋₈ cycloalkyl C₂₋₉ alkenyl, C₃₋₈ cycloalkyl C₂₋₉ alkynyl.

10. The isoquinolinone compound or the stereoisomer, the pharmaceutically acceptable salt, the solvate or the crystal thereof according to claim 1, is **characterized in that**, R₂ is selected from C₁₋₃ alkoxy, halogen, C₃₋₆ cycloalkyl, benzyl.

11. The isoquinolinone compound or the stereoisomer, the pharmaceutically acceptable salt, the solvate or the crystal thereof according to claim 1, is **characterized in that**, R₆ is selected from methyl, ethyl, isopropyl, butyl, isobutyl, methoxy methyl, methoxy ethyl, methoxy isopropyl, methoxy butyl, methoxy isobutyl, ethoxy methyl, ethoxy ethyl, ethoxy isopropyl, ethoxy butyl, ethoxy isobutyl, hydroxyl methyl, hydroxyl ethyl, hydroxyl isopropyl, hydroxyl butyl and hydroxyl isobutyl.

12. The isoquinolinone compound or the stereoisomer, the pharmaceutically acceptable salt, the solvate or the crystal thereof according to claim 1, is **characterized in that**, except for active hydrogens, all other hydrogen atoms can be independently replaced by deutrium.

13. The isoquinolinone compound or the stereoisomer, the pharmaceutically acceptable salt, the solvate or the crystal thereof according to claim 1, is **characterized in that**, the isoquinolinone compound is selected from the following compounds:

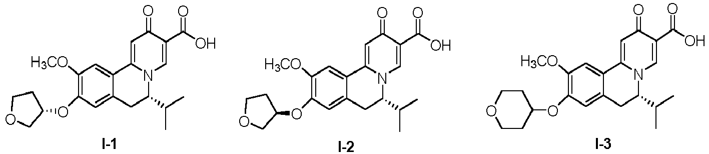

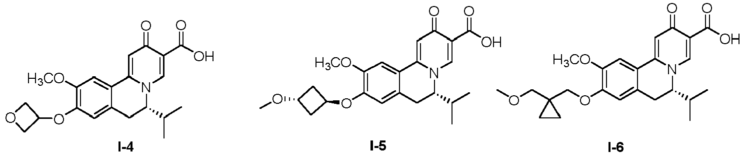

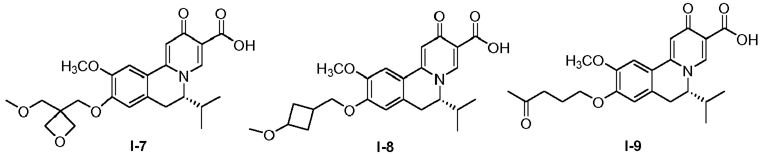

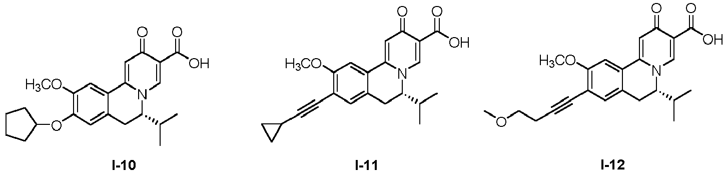

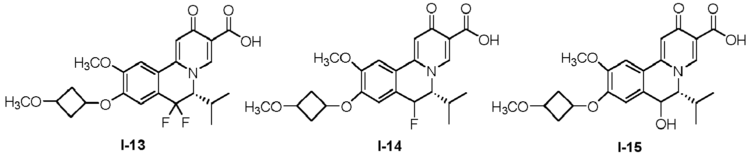

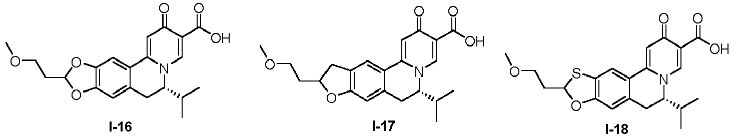

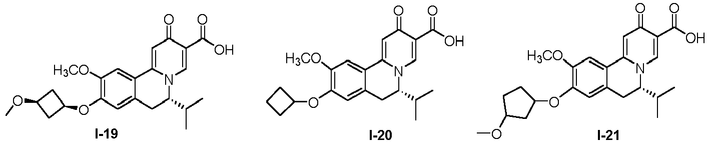

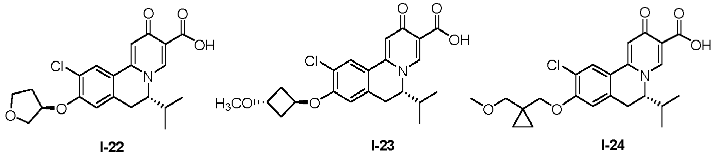

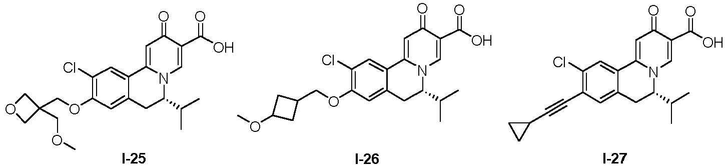

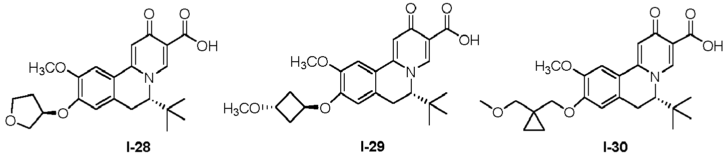

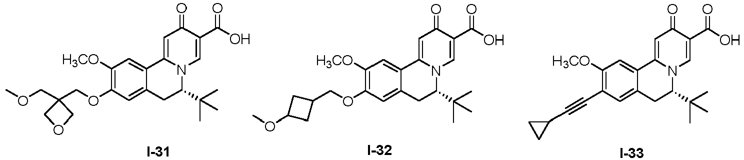

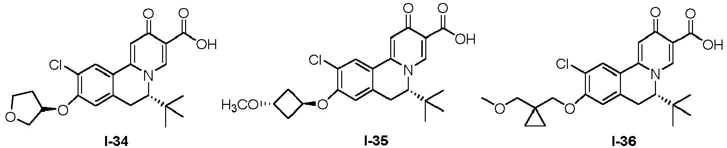

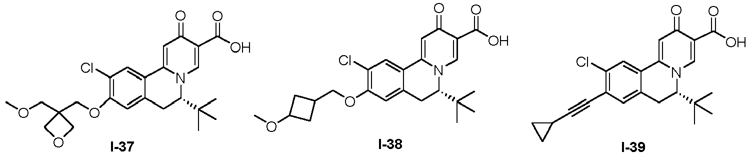

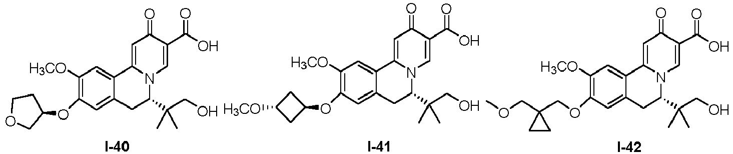

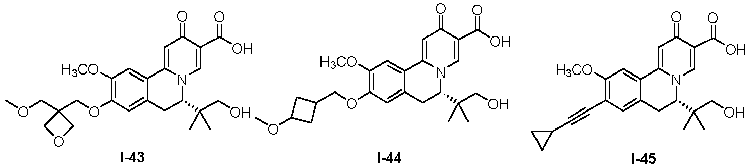

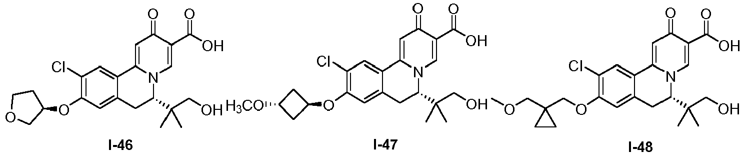

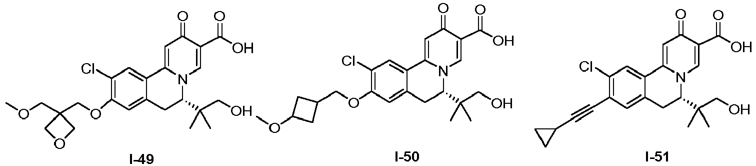

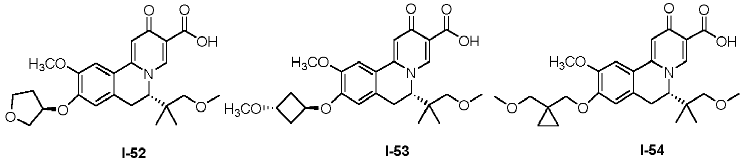

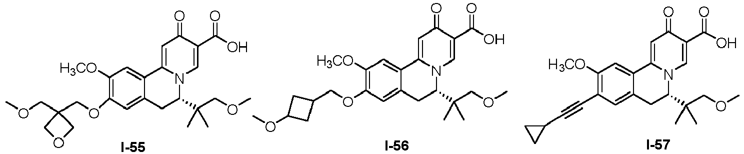

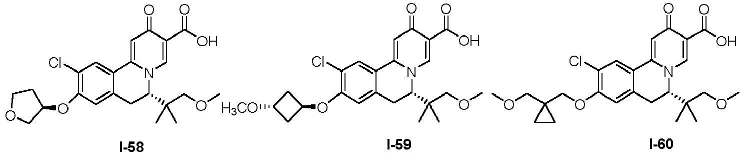

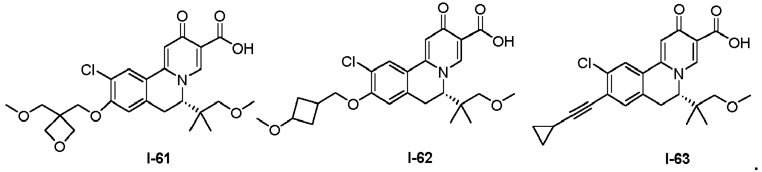

14. A pharmaceutical composition, is **characterized in that**, it contains the isoquinolinone compound shown in Formula (1) or the stereoisomer, the pharmaceutically acceptable salt, the solvate or the crystal thereof according to any one of claims 1 to 13, and a pharmaceutically acceptable carrier or excipient, and a dosage form of the pharmaceutical composition is preferably a tablet, capsule or injection.

15. The pharmaceutical composition according to claim 14, is **characterized in that**, the pharmaceutical composition is an antiviral pharmaceutical compositon, wherein it contains one or more therapeutic agents selected from : nucleoside drugs, ribavirin, interferons, HBV capsid inhibitors, cccDNA formation inhibitors, cccDNA epigenetic modifiers or hepatitis B RNAi drugs and TLR7 agonists.

16. Use of the isoquinolinone compound shown in Formula (1) or the stereoisomer, the pharmaceutically acceptable salt, the solvate or the crystal thereof according to claims 1 to 13 or a combination thereof with one or more therapeutic agents selected from nucleoside drugs, ribavirin, interferons, HBV capsid inhibitors, cccDNA formation inhibitors, cccDNA epigenetic modifiers, hepatitis B RNAi drugs or TLR7 agonists in the preparation of a medicament for preventing and/or treating virus infection diseases, and/or in the preparation of HVB surface antigen inhibitors and HVB DNA production inhibitors, the virus infection includes infection with HBV or HDV.

17. An intermediate for preparing the isoquinolinone compound shown in Formula (1) or the stereoisomer, the pharmaceutically acceptable salt, the solvate or the crystal thereof according to any one of claims 1 to 13, is **characterized in that**, the intermediate is shown in Formula (II): 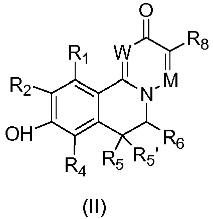 in Formula (II), R₁, R₂, R₄, R₅, R₅', R₆, R₈, W and N are as defined in the preceding claims.

18. The intermediate according to claim 17, is **characterized in that**, the intermediate shown in Formula (II) is Compound 10 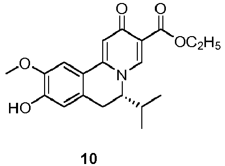 or an isomer or racemate thereof.

19. A process for preparing the isoquinolinone compound shown in Formula (I) or the stereoisomer, the pharmaceutically acceptable salt, the solvate or the crystal thereof according to any one of claims 1 to 13, is **characterized in that**, the process comprises employing the intermediate shown in Formula (II) according to claim 17 or 18, reacting the intermediate shown in Formula (II) with R_{A}OH, R_{A}OMs or R_{A}Br, wherein, when the reactant is R_{A}OH, the reaction is carried out using a Mitsunobu reactionin the presence of a dehydrating agent of triphenylphosphine and/or diisopropyl azodicarboxylate; when the reactant is R_{A}OMs or R_{A}Br, the reaction is an SN₂ reaction, and carried out in the presence of a base of potassium carbonate and/or cesium carbonate and a catalytic amount of KI.
